# EUROPEAN PATENT SPECIFICATION

(11) **EP 4 009 957 B1**
(45) Date of publication and mention of the grant of the patent: **11.10.2023**
(21) Application number: 20754702.7
(22) Date of filing: 06.08.2020
(51) Int. Cl.: A61K 9/16, A61K 9/20, A61K 38/22, A61K 38/26, A61P 3/04, A61P 3/10

(54) **SOLID COMPOSITION COMPRISING A PYY COMPOUND AND A SALT OF N-(8-(2-HYDROXYBENZOYL)AMINO)CAPRYLIC ACID**
FESTE ZUSAMMENSETZUNGEN MIT EINER PYY-VERBINDUNG UND EINEM SALZ VON N-(8-(2-HYDROXYBENZOYL)AMINO)CAPRYLSÄURE
COMPOSITION SOLIDE COMPORTANT UN COMPOSÉ PYY ET UN SEL D'ACIDE N-(8-(2-HYDROXYBENZOYL)AMINO)CAPRYLATE

(30) Priority: 07.08.2019 EP 19190571
(43) Date of publication of application: 15.06.2022
(73) Proprietor: Novo Nordisk A/S, 2880 Bagsværd (DK)
(72) Inventor: VEGGE, Andreas, 2880 Bagsværd (DK); WULFF, Birgitte, Schjellerup, 2880 Bagsværd (DK); NISSEN, Birgitte, 2880 Bagsværd (DK); ØSTERGAARD, Søren, 2880 Bagsværd (DK); PEDERSEN, Betty Lomstein, 2880 Bagsværd (DK)
(86) International application number: PCT/EP2020/072137
(87) International publication number: WO 2021/023817

(56) References cited:
- WO-A1-2012/080471
- WO-A1-2013/139694
- WO-A1-2016/198682
- WO-A2-2004/104018
- BEGLINGERL C. ET AL.: "Pharmacokinetics and Pharmacodynamic Effects of Oral GLP-1 and PYY3-36: A Proof-of-concept Study in Healthy Subjects", CLINICAL PHARMACOLOGY AND THERAPEUTICS, NATURE PUBLISHING GROUP, US, vol. 84, no. 4, 1 October 2008 (2008-10-01), pages 468-474, XP008149454, ISSN: 0009-9236, DOI: 10.1038/CLPT.2008.35 [retrieved on 2008-03-26]
- STEINERT R.E. ET AL.: "Oral administration of glucagon-like peptide 1 or peptide YY 3-36 affects food intake in healthy male subjects", AM. J. CLIN. NUTR., vol. 92, 2010, pages 810-817, XP002797377,

## Description

### TECHNICAL FIELD

The present invention relates to solid compositions comprising a Peptide tyrosine tyrosine (PYY) compound and a salt of N-(8-(2-hydroxybenzoyl)amino)caprylic acid and their use in medicine.

### BACKGROUND

PYY is released during a meal from L-cells in the distal small intestine and the colon. PYY is known to have peripheral effects in the gastrointestinal (GI) tract and also act centrally as a satiety signal. PYY is naturally secreted as a 36 amino acid peptide (PYY(1-36)) with a C-terminal amide but is cleaved to PYY(3-36) which constitutes approximately 50% of the circulating PYY. The enzyme responsible for the degradation is dipeptidyl peptidase IV (DPPIV). PYY(3-36) is rapidly eliminated by proteases and other clearance mechanisms. The half-life of PYY(3-36) has been reported to be <30 minutes in pigs. Thus, PYY displays suboptimal pharmacokinetic properties, meaning that the peptide has to be administered at least twice daily.

Whereas PYY(1-36) activates Y1, Y2 and Y5 receptors with very little selectivity and the Y4 receptor slightly less, the DPPIV processed PYY(3-36) displays increased selectivity for the Y2 receptor over Y1, Y4 and Y5 receptors, albeit some Y1 and Y5 affinity is retained. Y2 receptor activation is known to decrease appetite and food intake whereas Y1 and Y5 receptor activation leads to an increase in appetite and food intake. Furthermore, Y1 and Y5 receptor activation may lead to an increase in blood pressure.

PYY(3-36) has been suggested for use in the treatment of obesity and associated diseases based on the demonstrated effects of certain of these peptides in animal models and in man, and on the fact that obese people have low basal levels of PYY as well as lower meal responses of this peptide. Furthermore, Y2 receptor agonists have been demonstrated to have anti-secretory and pro-absorptive effects in the gastro-intestinal (GI) tract. The potential use of Y2 receptor agonists in the treatment of a number of GI disorders has been suggested.

Based on demonstrated effects in e.g. Zucker rats and Diet-Induced Obese (DIO) mice Y2 selective PYY(3-36) analogues have a positive effect on glucose metabolism and are thus suggested to be used for the treatment of diabetes. WO2011/058165, WO2015/071355 and WO2016/198682 disclose examples of Y2 selective receptor agonists with protracted pharmacokinetic properties.

PYY(3-36) has been administered in combination with other peptides e.g. in combination with GLP-1(7-36) and shown promising results for treatment of obesity and diabetes (Schmidt et al., Am J Physiol Endocrinol Metab, 306: E1248-E1256, 2014).

Human PYY and analogues thereof have a low oral bioavailability. Human PYY and analogues thereof can only be detected in plasma after oral administration if formulated with certain absorption enhancers in a specific amount.

Steinert et al. (Am J Clin Nutr, Oct 2010; 92, 810-817) discloses oral administration of a tablet comprising PYY(3-36) and 150 mg sodium N-(8-(2-hydroxybenzoyl)amino)caprylate (SNAC), and also a tablet comprising a combination of GLP-1(7-36) and PYY(3-36) with 150 mg SNAC. WO2004/104018 and WO2006/017251 describe oral administration of PYY(3-36) using SNAC as delivery agent in the form of a tablet or for buccal administration, respectively. GLP-1 receptor agonist semaglutide has been formulated with SNAC to obtain tablets with oral bioavailability (WO2012/080471 and WO2013/139694).

Despite these findings there is still a need for an optimized pharmaceutical composition for oral administration, the composition comprising a PYY compound optionally in combination with a GLP-1 receptor agonist.

### SUMMARY

In one aspect, the present invention relates to a composition comprising a PYY compound and an absorption enhancer or delivery agent. In one embodiment, the excipients of the composition according to the invention includes a very high content of the delivery agent and a minimal content of further excipients as described herein. The provided compositions display an accelerated dissolution enabling a faster uptake of the active pharmaceutical ingredient.

Oral administration of therapeutic peptides is challenging due to the rapid degradation of such peptides in the GI system.

Described herein are pharmaceutical compositions providing accelerated dissolution and absorption of the PYY compound by oral administration. Based on previous data obtained for semaglutide, an improved exposure of the PYY compound by oral administration is therefore foreseen. The inventors have surprisingly found that the dissolution of the PYY compound increases when the excipients of the composition comprises a very high content of the absorption enhancer and a minimal content of any further excipients.

In one aspect, the invention relates to a composition wherein the weight ratio of the delivery agent relative to the total composition, or in particular, relative to the other excipients of the composition, is very high.

In one embodiment, the invention relates to a pharmaceutical composition comprising a PYY compound, a delivery agent and/or absorption enhancer such as SNAC, wherein the delivery agent/absorption enhancer constitutes at least 90 percent (w/w) of the excipients of the composition.

In one embodiment, the invention relates to a pharmaceutical composition comprising a PYY compound, a delivery agent and/or absorption enhancer such as SNAC, wherein the delivery agent/absorption enhancer constitutes at least 60 percent (w/w) of the composition.

In an additional embodiment, the composition further includes a lubricant.

In one aspect, the invention relates to the composition of the invention for use in medicine, e.g. for the treatment of diabetes and/or obesity, wherein said composition is administered orally.

In one aspect, the invention relates to a method of treating diabetes or obesity comprising administering the composition as defined herein to a patient in need thereof, wherein said composition is a tablet and is administered orally.

### BRIEF DESCRIPTION OF DRAWINGS

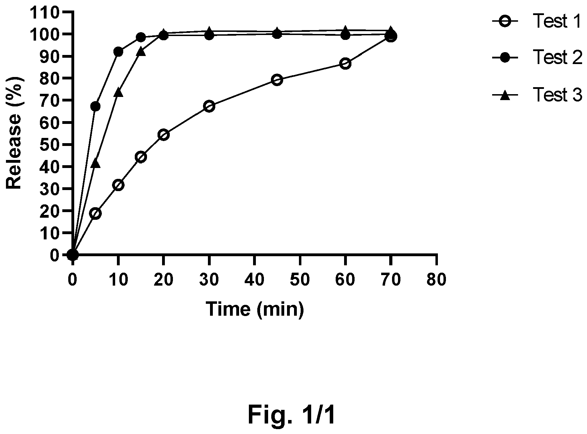

Fig. 1 shows fast dissolution (release) of PYY compound 4 of test composition 2 and test composition 3 as compared to test composition 1.

### DESCRIPTION

In one aspect, the invention relates to a composition comprising a PYY compound and an absorption enhancer or delivery agent. The composition may be in the form suitable for oral administration, such as a tablet, sachet or capsule. In one embodiment, the composition is an oral composition, or a pharmaceutical composition, such as an oral pharmaceutical composition.

In one embodiment, the composition according to the invention includes a high content of the delivery agent and a minimal content of further excipients as described herein below. The provided compositions display an accelerated dissolution and thereby enables fast and efficient uptake of the active pharmaceutical ingredient.

### PYY compounds

The term "hPYY(1-36)" as used herein refers to the human Peptide YY, the sequence of which is included in the sequence listing as SEQ ID NO:1. The peptide having the sequence of SEQ ID NO:1 may also be designated native hPYY.

The term "PYY compound" as used herein refers to a peptide, or a compound, which is a variant of hPYY(1-36). The term "PYY compound" as used herein may also refer to a peptide, or a compound, which is a variant of hPYY(3-36) (SEQ ID NO:2). The term "PYY compound" as used herein may also refer to a peptide, or a compound, which is a variant of hPYY(4-36) (SEQ ID NO: 29). In some embodiments, the term "variant" refers to a compound which comprises one or more amino acid substitutions, deletions, additions and/or insertions.

The C-terminal of the PYY compounds comprised in the present composition is an amide, as is the C-terminal of native hPYY(1-36) (SEQ ID NO:1), hPYY(3-36) (SEQ ID NO:2) and hPYY(4-36) (SEQ ID NO: 29), respectively.

The PYY compounds comprised in the present composition can be PYY analogues and/or derivatives thereof.

The term "PYY analogue" is used for PYY compounds, where at least one amino acid modification in the backbone is present as compared to hPYY(1-36).

The term "PYY derivative" is used for PYY compounds comprising at least one non-amino acid substituent covalently attached. Thus, a PYY derivative is a derivative of a PYY analogue and thus, a PYY compound comprising at least one amino acid modification in the backbone and at least one non-amino acid substituent covalently attached.

The PYY compounds comprised in the present composition may comprise up to 10 amino acid modifications as compared to hPYY(3-36) (SEQ ID NO:2).

The term "amino acid modification" used throughout this application is used in the meaning of a modification to an amino acid as compared to hPYY(3-36). This modification can be the result of a deletion of an amino acid, addition of an amino acid, or substitution of one amino acid with another.

In one aspect, the PYY compound comprised in the present composition, is described in example 1 of WO2016/198682 (compounds 1-63, 65-71) and their biological data as described therein potency (example 2), binding affinity (example 3), half-life (example 4), and their pharmacodynamic data (effect on blood glucose and food intake, example 5).

In one aspect, the PYY compound comprised in the present composition may comprise i) lysine at the position corresponding to position 7 or 10 of hPYY(1-36) (SEQ ID NO:1); ii) tryptophan at the position corresponding to position 30 of hPYY(1-36) (SEQ ID NO:1); iii) leucine at the position corresponding to position 31 of hPYY(1-36) (SEQ ID NO:1); and iv) tyrosine at the position corresponding to position 28 of hPYY(1-36) (SEQ ID NO:1), meaning that the PYY compounds of this aspect may comprise up to 6 amino acid modifications as compared to hPYY(3-36) in addition to these modification in the positions corresponding to positions 7, 30, 28, and 31 of hPYY(1-36) (SEQ ID NO:1).

In another aspect, the PYY compounds comprised in the present composition may comprise i) lysine at the position corresponding to position 7 or 10 of hPYY(1-36) (SEQ ID NO:1); ii) tryptophan at the position corresponding to position 30 of hPYY(1-36) (SEQ ID NO:1); iii) leucine at the position corresponding to position 31 of hPYY(1-36) (SEQ ID NO:1); iv) tyrosine at the position corresponding to position 28 of hPYY(1-36) (SEQ ID NO:1); and v) isoleucine at the position corresponding to position 22 of hPYY(1-36) (SEQ ID NO:1), meaning that the PYY compounds of this aspect may comprise up to 5 amino acid modifications as compared to hPYY(3-36) in addition to these modification in the positions corresponding to positions 7, 30, 22, 28, and 31 of hPYY(1-36) (SEQ ID NO:1).

As an example, [Arg4,Lys7,Gln18,Tyr28,Trp30,Leu31]hPYY(3-36) comprises 6 amino acid substitutions as compared to hPYY(3-36). As another example, [Arg4,Lys7,Gln18,Tyr28,Trp30,Leu31]hPYY(4-36) comprises 6 amino acid substitutions and 1 deletion as compared to hPYY(3-36), meaning that this compound has 7 amino acid modifications as compared to hPYY(3-36).

PYY compounds comprised in the present composition may be described by reference to i) the number of the amino acid residue in hPYY(1-36) (SEQ ID NO:1) which corresponds to the amino acid residue which is changed (i.e., the corresponding position in hPYY(1-36), and to ii) the actual change.

The expressions "a position equivalent to" or "corresponding position" are used to characterise the site of change in a variant PYY sequence by reference to hPYY(1-36).

In general throughout the application, when referring to a particular position of a PYY analogue, the position referred to is the position of the PYY analogue corresponding to that particular position of hPYY(1-36).

In the sequence listing, the first amino acid residue of a given sequence is assigned no. 1. This means that e.g. the first amino acid residue of hPYY(3-36), which is isoleucine, is assigned no. 1 in the sequence listings. Throughout this application however, this position is referred to as the position corresponding to position 3 of hPYY(1-36).

The expression used throughout this application, that a PYY compound comprises a particular amino acid at a position corresponding to a certain position of hPYY(1-36), means that the native amino acid in that position has been replaced with that particular amino acid.

The following is a non-limiting example of suitable analogue nomenclature.

[Lys7,Tyr28,Trp30,Leu31]hPYY(3-36) designates an analogue of the human PYY(1-36), wherein the naturally occurring alanine in position 7 has been substituted with lysine, the naturally occurring leucine in position 28 has been substituted with tyrosine, the naturally occurring leucine in position 30 has been substituted with tryptophan, the naturally occurring valine in position 31 has been substituted with leucine, and tyrosine and proline in position 1 and 2, respectively, have been deleted. Likewise, [Lys7,Tyr28,Trp30,Leu31]hPYY(3-36) can also be said to designate an analogue of the human PYY(3-36), wherein the naturally occurring alanine in position 7 has been substituted with lysine, the naturally occurring leucine in position 28 has been substituted with tyrosine, the naturally occurring leucine in position 30 has been substituted with tryptophan, and the naturally occurring valine in position 31 has been substituted with leucine.

The following is a non-limiting example of suitable nomenclature for a derivative of a PYY analogue. N{alpha-4}-(3-Methylbutanoyl)-N{Epsilon-7}-[(4S)-4-carboxy-4-(17-carboxyheptadecanoylamino)butanoyl]-[Arg4,Lys7,GIn18,Tyr28,Trp30,Leu31]hPYY(4-36) designates a derivative of an analogue of hPYY(4-36), wherein [Arg4,Lys7,Gln18,Tyr28,Trp30,Leu31] designate the amino acid changes as compared to human PYY(4-36) with the numbers referring to the corresponding positions of PYY(1-36), and wherein the substituent 3-methylbutanoyl is attached to the alpha amino group of the N-terminal amino acid residue, and the substituent [(4S)-4-carboxy-4-(17-carboxyheptadecanoylamino)butanoyl] is attached to the epsilon amino group of the lysine in the position corresponding to position 7 in hPYY(1-36).

Amino acid residues may be identified by their full name, their one-letter code, and/or their three-letter code. These three ways are fully equivalent.

Analogues "comprising" certain specified changes may comprise further changes, when compared to hPYY(1-36). In one aspect, the analogue "has" the specified changes.

### PYY analogues

A PYY analogue is a PYY compound in which a number of amino acid residues have been modified when compared to hPYY(1-36) or hPYY(3-36). These modifications include substitutions, insertions, and/or deletions, alone or in combination.

In a specific aspect, the PYY analogues comprised in the composition of the invention include one or more modifications of a "non-essential" amino acid residue. In the context of the invention, a "non-essential" amino acid residue is a residue that can be altered, i.e., deleted or substituted in the human PYY amino acid sequence without abolishing or substantially reducing the activity of the PYY analogue towards the Y2 receptor.

In one aspect amino acids may be substituted by conservative substitution. The term "conservative substitution" as used herein denotes that one or more amino acids are replaced by another, biologically similar residue. Examples include substitution of amino acid residues with similar characteristics, e.g. small amino acids, acidic amino acids, polar amino acids, basic amino acids, hydrophobic amino acids and aromatic amino acids.

In one aspect, the PYY analogues comprised in the composition of the invention may comprise substitutions of one or more unnatural and/or non-amino acids, e.g., amino acid mimetics, into the sequence of PYY.

In one aspect, the PYY analogues comprised in the composition of the invention may have one or more amino acid residues deleted from the amino acid sequence of human PYY, alone or in combination with one or more insertions or substitutions.

In one aspect, the PYY analogues comprised in the composition of the invention may have one or more amino acid residues inserted into the amino acid sequence of human PYY, alone or in combination with one or more deletions and/or substitutions.

In one aspect, the PYY analogues of the invention may include insertions of one or more unnatural amino acids and/or non-amino acids into the sequence of PYY.

The term "amino acid" includes proteinogenic (or coded or natural) amino acids (amongst the 20 standard amino acids), as well as non-proteinogenic (or non-coded or non-natural) amino acids. Proteinogenic amino acids are those which are naturally incorporated into proteins. The standard amino acids are those encoded by the genetic code. Non-proteinogenic amino acids are either not found in proteins, or not produced by standard cellular machinery (e.g., they may have been subject to post-translational modification). Non-limiting examples of non-proteinogenic amino acids are the D-isomers of the proteinogenic amino acids. One example of a D-isomer of a proteinogenic amino acid is the D-isomer of aspartic acid, which can also be written as D-Asp.

In what follows, all amino acids of the PYY compound for which the optical isomer is not stated is to be understood to mean the L-isomer (unless otherwise specified).

### PYY derivatives

The term "derivative" as used herein in the context of a PYY compound or analogue means a chemically modified PYY peptide, in which one or more substituents have been covalently attached to the peptide.

In one aspect of the invention, the substituent may be an N-terminal substituent.

Also or alternatively, in one aspect, the substituent may be a modifying group or alternatively, referred to as a protracting moiety.

### N-terminal substituent

In one aspect of the invention, the PYY compound comprises a substituent covalently attached to the alpha-amino group in the amino acid residue in the N-terminus of the PYY compound. In one aspect, the amino acid residues in the positions corresponding to positions 1-3 of hPYY(1-36) are absent, and the N-terminal substituent is covalently attached to the amino acid residue in the position corresponding to position 4 of hPYY(1-36).

In one aspect, the N-terminal substituent is an alkanoyl group. In one aspect, the N-terminal substituent is an alkanoyl group comprising up to 12 carbon atoms. In another aspect, the N-terminal substituent is an alkanoyl group comprising up to 6 carbon atoms. In another aspect, the N-terminal substituent is selected form 3-methylbutanoyl or acetyl.

### Modifying group

In one aspect, the PYY compound comprises a modifying group covalently attached to the amino acid residue in the position corresponding to position 7 or 10 of hPYY(1-36). In one further aspect, the substituent or modifying group is capable of forming non-covalent conjugates with proteins, thereby promoting the circulation of the derivative with the blood stream, and also having the effect of protracting the time of action of the derivative, due to the fact that the conjugate of the PYY derivative and albumin is only slowly removed by renal clearance. Thus, the substituent, or modifying group, as a whole may also be referred to as a protracting moiety.

The modifying group may be covalently attached to a lysine residue of the PYY peptide by acylation, i.e., via an amide bond formed between a carboxylic acid group of the modifying group and the epsilon amino group of the lysine residue. The amino group of lysine could also be coupled to an aldehyde of the modifying group by reductive amination. In another aspect, the thiol group of cysteine could by coupled to a maleiimido group of the modifying group by Michael addition or coupled to the chloro- or iodoacetyl group of the modifying group by nucleophilic substitution.

In one aspect, the modifying group is covalently attached to a lysine residue in a position corresponding to position 7 or 10 of hPYY(1-36) by acylation, i.e. via an amide bond formed between a carboxylic acid group of the modifying group and the epsilon amino group of the lysine residue.

In some embodiments, the PYY compound comprised in the composition of the invention is a PYY derivative selected from compounds 1-63, 65-71 in WO2016/198682 (Example 1) or as enclosed under embodiments herein as compounds 1-63, 65-71 and the peptide backbones as SEQ ID NO: 3-28.

### Delivery agent

The delivery agent used in the composition of the present invention is a salt of N-(8-(2-hydroxybenzoyl)amino)caprylic acid (NAC) and is included as an excipient. The structural formula of N-(8-(2-hydroxybenzoyl)amino)caprylate is shown in formula (I).

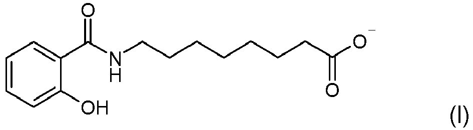

In some embodiments, the salt of N-(8-(2-hydroxybenzoyl)amino)caprylic acid comprises one monovalent cation, two monovalent cations or one divalent cation. In some embodiments, the salt of N-(8-(2-hydroxybenzoyl)amino)caprylic acid is selected from the group consisting of the sodium salt, potassium salt and/or calcium salt of N-(8-(2-hydroxybenzoyl)amino)caprylic acid. In one embodiment, the salt of N-(8-(2-hydroxybenzoyl)amino)caprylic acid is selected from the group consisting of the sodium salt, potassium salt and/or the ammonium salt. In one embodiment, the salt of N-(8-(2-hydroxybenzoyl)amino)caprylic acid is the sodium salt or the potassium salt. In one embodiment, the salt of N-(8-(2-hydroxybenzoyl)amino)caprylic acid is the sodium salt or the ammonium salt. Salts of N-(8-(2-hydroxybenzoyl)amino)caprylate may be prepared using the method described in e.g. WO96/030036, WO00/046182, WO01/092206 or WO2008/028859.

The salt of N-(8-(2-hydroxybenzoyl)amino)caprylic acid may be crystalline and/or amorphous. In some embodiments, the delivery agent comprises the anhydrate, monohydrate, dihydrate, trihydrate, a solvate or one third of a hydrate of the salt of N-(8-(2-hydroxybenzoyl)amino)caprylic acid as well as combinations thereof. In some embodiments, the delivery agent is a salt of N-(8-(2-hydroxybenzoyl)amino)caprylic acid as described in WO2007/121318.

In some embodiments, the delivery agent is sodium N-(8-(2-hydroxybenzoyl)amino)caprylate (referred to as "SNAC" herein), also known as sodium 8-(salicyloylamino)octanoate.

### Combination with GLP-1 receptor agonist

In some embodiments, the composition of the invention further comprises a GLP-1 receptor agonist in combination with the PYY compound. The term "GLP-1 receptor agonist" as used herein refers to a compound, which fully or partially activates the human GLP-1 receptor. The term GLP-1 receptor agonist as well as the specific GLP-1 receptor agonists described herein are meant to encompass also salt forms hereof.

It follows that the GLP-1 receptor agonist should display "GLP-1 activity" which refers to the ability of the compound, i.e. a GLP-1 analogue or a compound comprising a GLP-1 analogue, to bind to the GLP-1 receptor and initiate a signal transduction pathway resulting in insulinotropic action or other physiological effects as is known in the art. In some embodiments, the "GLP-1 receptor agonist" binds to a GLP-1 receptor, e.g. with an affinity constant (K_{D}) or activate the receptor with a potency (EC₅₀) of below 1 µM, e.g. below 100 nM as measured by methods known in the art (see e.g. WO 98/08871) and exhibits insulinotropic activity, where insulinotropic activity may be measured *in vivo* or *in vitro* assays known to those of ordinary skill in the art. For example, the GLP-1 receptor agonist may be administered to an animal with increased blood glucose (e.g. obtained using an Intravenous Glucose Tolerance Test (IVGTT). A person skilled in the art will be able to determine a suitable glucose dosage and a suitable blood sampling regime, e.g. depending on the species of the animal, for the IVGTT) and measure the plasma insulin concentration over time. Suitable assays have been described in such as WO2015/155151.

In some embodiments, the GLP-1 receptor agonist is a GLP-1 analogue, optionally comprising one substituent. The term "GLP-1 analogue" as used herein referring to a peptide, or a compound, which is a variant of the human Glucagon-like Peptide-1 (GLP-1(7-37)). GLP-1(7-37) has the sequence HAEGTFTSDV SSYLEGQAAKEFIAWLVKGRG (SEQ ID NO: 31). GLP-1(7-36) has the sequence HAEGTFTSDV SSYLEGQAAKEFIAWLVKGR (SEQ ID NO: 30). In some embodiments, the term "variant" refers to a compound which comprises one or more amino acid substitutions, deletions, additions and/or insertions.

In one embodiment, the C-terminal of the GLP-1 receptor agonist is an amide. In some embodiments, the GLP-1 receptor agonist is GLP-1(7-37)amide or GLP-1(7-36)amide.

In some embodiments, the GLP-1 receptor agonist comprises a substituent, wherein the substituent is [2-(2-{2-[2-(2-{2-[(S)-4-carboxy-4-(17-carboxyheptadecanoylamino) butyrylamino]ethoxy}ethoxy)acetylamino] ethoxy}ethoxy)acetyl]. In some embodiments, the substituent is [2-(2-{2-[2-(2-{2-[(S)-4-carboxy-4-({trans-4-[(19-carboxynonadecanoylamino)methyl]cyclohexanecarbonyl} amino)butyrylamino]ethoxy}ethoxy)acetylamino]ethoxy}ethoxy)acetyl].

In some embodiments, the GLP-1 receptor agonist is semaglutide, also known as *N-*epsilon26-[2-(2-{2-[2-(2-{2-[(S)-4-carboxy-4-(17-carboxyheptadecanoylamino) butyrylamino]ethoxy}ethoxy)acetylamino]ethoxy}ethoxy)acetyl][Aib8,Arg34]GLP-1 (7-37) (SEQ ID NO: 32), which may be prepared as described in WO2006/097537, Example 4 and has the following structure:

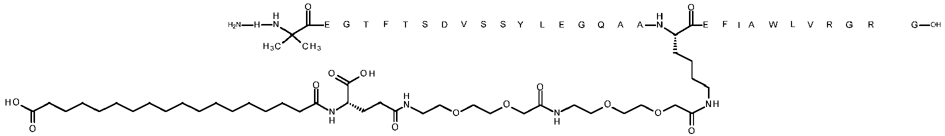

In one embodiment, the GLP-1 receptor agonist is "GLP-1 agonist A" which is Diacylated [Aib8,Glu22,Arg26, Lys27, Glu30,Arg34, Lys36]-GLP-1-(7 -37)-peptidyl-Glu-Gly (SEQ ID NO. 33) as shown in Example 31 of WO2012/140117 and named N-epsilon27-[2-[2-[2-[[2-[2-[2-[[(4S)-4-carboxy-4-[10-(4-carboxyphenoxy)decanoylamino]butanoyl]amino] ethoxy]ethoxy]acetyl]amino]ethoxy]ethoxy]-acetyl], N-epsilon36-[2-[2-[2-[[2-[2-[2-[[(4S)-4-carboxy-4-[10-(4-carboxyphenoxy)decanoylamino]-butanoyl]amino]ethoxy]ethoxy]acetyl]amino]ethoxy]ethoxy]acetyl]-[Aib8,Glu22,Arg26,Lys27, Glu30,Arg34,Lys36]-GLP-1-(7-37)-peptidyl-Glu-Gly and has the following structure:

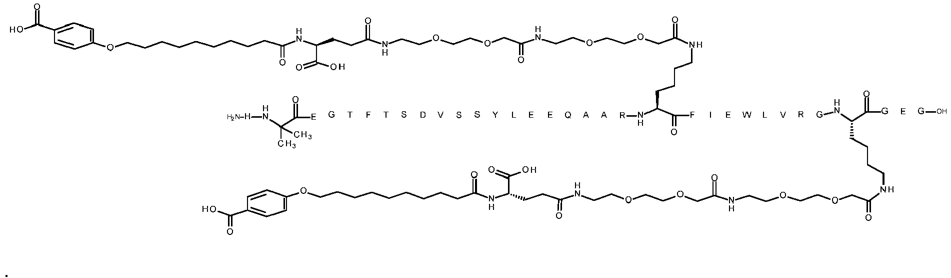

Semaglutide and GLP-1 agonist A, have been shown to be orally available when formulated in a SNAC formulation as described in PCT application no. PCT/EP2019/052487.

In general, the term "GLP-1 receptor agonist" is meant to encompass the GLP-1 receptor agonist and any pharmaceutically acceptable salt, amide, or ester thereof. In some embodiments, the composition comprises the GLP-1 receptor agonist or a pharmaceutically acceptable salt, amide, or ester thereof. In some embodiments, the composition comprises the GLP-1 receptor agonist and one or more pharmaceutically acceptable counter ions.

In some embodiments, the GLP-1 receptor agonist is selected from one or more of the GLP-1 receptor agonists mentioned in WO93/19175, WO96/29342, WO98/08871, WO99/43707, WO99/43706, WO99/43341, WO99/43708, WO2005/027978, WO2005/058954, WO2005/058958, WO2006/005667, WO2006/037810, WO2006/037811, WO2006/097537, WO2006/097538, WO2008/023050, WO2009/030738, WO2009/030771 and WO2009/030774.

### Composition

The composition or pharmaceutical composition of the present invention is a solid or dry composition suited for administration by the oral route as described further herein below.

In some embodiments, the composition comprises at least one pharmaceutically acceptable excipient. The term **"excipient"** as used herein broadly refers to any component other than the active therapeutic ingredient(s) or active pharmaceutical ingredient(s) (API(s)). An excipient may be a pharmaceutically inert substance, an inactive substance, and/or a therapeutically or medicinally none active substance.

The excipients may serve various purposes, e.g. as a carrier, vehicle, filler, binder, lubricant, glidant, disintegrant, flow control agent, crystallization inhibitors, solubilizer, stabilizer, colouring agent, flavouring agent, surfactant, emulsifier or combinations of thereof and/or to improve administration, and/or absorption of the therapeutically active substance(s) or active pharmaceutical ingredient(s). As described herein the salt of N-(8-(2-hydroxybenzoyl)amino)caprylic acid is an excipient acting as a delivery agent. The amount of each excipient used may vary within ranges conventional in the art. Techniques and excipients which may be used to formulate oral dosage forms are described in Handbook of Pharmaceutical Excipients, 8th edition, Sheskey et al., Eds., American Pharmaceuticals Association and the Pharmaceutical Press, publications department of the Royal Pharmaceutical Society of Great Britain (2017); and Remington: the Science and Practice of Pharmacy, 22nd edition, Remington and Allen, Eds., Pharmaceutical Press (2013).

In some embodiments, the excipients may be selected from **binders,** such as polyvinyl pyrrolidone (povidone), etc.; **fillers** such as cellulose powder, microcrystalline cellulose, cellulose derivatives like hydroxymethylcellulose, hydroxyethylcellulose, hydroxypropylcellulose and hydroxy-propylmethylcellulose, dibasic calcium phosphate, corn starch, pregelatinized starch, etc.; **lubricants** and/or **glidants** such as stearic acid, magnesium stearate, sodium stearylfumarate, glycerol tribehenate, etc.; **flow control agents** such as colloidal silica, talc, etc.; **crystallization inhibitors** such as povidone, etc.; **solubilizers** such as pluronic, povidone, etc.; **colouring agents,** including dyes and pigments such as iron oxide red or yellow, titanium dioxide, talc, etc.; **pH control agents** such as citric acid, tartaric acid, fumaric acid, sodium citrate, dibasic calcium phosphate, dibasic sodium phosphate, etc.; **surfactants** and **emulsifiers** such as pluronic, polyethylene glycols, sodium carboxymethyl cellulose, polyethoxylated and hydrogenated castor oil, etc.; and mixtures of two or more of these excipients and/or adjuvants.

The composition may comprise a **binder,** such as povidone; starches; celluloses and derivatives thereof, such as microcrystalline cellulose, e.g., Avicel PH from FMC (Philadelphia, PA), hydroxypropyl cellulose hydroxylethyl cellulose and hydroxylpropylmethyl cellulose METHOCEL from Dow Chemical Corp. (Midland, MI); sucrose; dextrose; corn syrup; polysaccharides; and gelatine. The **binder** may be selected from the group consisting of dry binders and/or wet granulation binders. Suitable dry binders are, e.g., cellulose powder and microcrystalline cellulose, such as Avicel PH 102 and Avicel PH 200. In some embodiments the composition comprises Avicel, such as Avicel PH 102. Suitable binders for wet granulation or dry granulation are corn starch, polyvinyl pyrrolidone (povidone), vinylpyrrolidone-vinylacetate copolymer (copovidone) and cellulose derivatives like hydroxymethylcellulose, hydroxyethylcellulose, hydroxypropylcellulose and hydroxyl-propylmethylcellulose. In some embodiments the composition comprises povidone.

In some embodiments, the composition comprises a **filler,** which may be selected from lactose, mannitol, erythritol, sucrose, sorbitol, calcium phosphate, such as calciumhydrogen phosphate, microcrystalline cellulose, powdered cellulose, confectioner's sugar, compressible sugar, dextrates, dextrin and dextrose. In some embodiments, the composition comprises microcrystalline cellulose, such as Avicel PH 101, Avicel PH 102 or Avicel PH 200.

In some embodiments, the composition comprises a lubricant and/or a glidant. In some embodiments, the composition comprises a **lubricant** and/or a **glidant,** such as talc, magnesium stearate, calcium stearate, zinc stearate, glyceryl behenate, glyceryl dibehenate, behenoyl polyoxyl-8 glycerides, polyethylene oxide polymers, sodium lauryl sulfate, magnesium lauryl sulfate, sodium oleate, sodium stearyl fumarate, stearic acid, hydrogenated vegetable oils, silicon dioxide and/or polyethylene glycol etc. In some embodiments, the composition comprises magnesium stearate or glyceryl dibehenate (such as the product Compritol^{®} 888 ATO which consists of mono-, di- and triesters of behenic acid (C22) with the diester fraction being predominant).

In some embodiments, the composition comprises a **disintegrant,** such as sodium starch glycolate, polacrilin potassium, sodium starch glycolate, crospovidon, croscarmellose, sodium carboxymethylcellulose or dried corn starch.

The composition may comprise one or more **surfactants,** for example a surfactant, at least one surfactant, or two different surfactants. The term "surfactant" refers to any molecules or ions that are comprised of a water-soluble (hydrophilic) part, and a fat-soluble (lipophilic) part. The surfactant may e.g. be selected from the group consisting of anionic surfactants, cationic surfactants, nonionic surfactants, and/or zwitterionic surfactants.

As shown in the examples herein, the compositions of the invention have a very high content of the delivery agent. This very high content can be defined relative to the full content of the tablets including also the active pharmaceutical ingredient (i.e. the PYY compound optionally in combination with a GLP-1 receptor agonist) or alternatively relative to the total content of excipients excluding the active pharmaceutical ingredient(s). The description here below also refers to compositions consisting of specific ingredients, the PYY compound, excipients, and optionally a GLP-1 receptor agonist, the term consisting is to be understood to never the less encompass trace amounts of any substance with no effect on the function of the composition, which may also be referred to as consisting essential of. Such substances can be impurities remaining in preparation of the PYY compound optionally from the GLP-1 receptor agonist or from the production of the salt of NAC or minimal amounts (below 1 %) of any pharmaceutical acceptable excipient that do not affect the quality or absorption of the formulation.

In one embodiment, the pharmaceutical composition comprises
a) a PYY compound and
b) a salt of N-(8-(2-hydroxybenzoyl)amino)caprylic acid (NAC)
wherein said salt of N-(8-(2-hydroxybenzoyl)amino)caprylic acid (NAC) constitutes at least or above 60 percent (w/w) of the composition.

In further such embodiments, the salt of NAC constitutes above 70 percent (w/w), such as above 75 percent (w/w), such as above 80 percent (w/w), such as above 85 percent (w/w), such as above 90 percent (w/w) of said composition.

In further such embodiments, the salt of NAC constitutes at least 70 percent (w/w), such as at least 75 percent (w/w), such as at least 80 percent (w/w), such as at least 85 percent (w/w), such as at least 90 percent (w/w) of said composition.

In one embodiment, the pharmaceutical composition comprises
a) a PYY compound and
b) a salt of N-(8-(2-hydroxybenzoyl)amino)caprylic acid (NAC),
wherein said salt of N-(8-(2-hydroxybenzoyl)amino)caprylic acid (NAC) constitutes at least 90 percent (w/w) of the excipients of the composition.

In further such embodiments, the salt of NAC constitutes at least at least 91 percent (w/w), such as at least 92 percent (w/w), such as at least 93 percent (w/w), such as at least 94 percent (w/w), such as at least 95 percent (w/w) of the excipients of the composition.

In one embodiment, the pharmaceutical composition consists of
c) a PYY compound and
d) a salt of N-(8-(2-hydroxybenzoyl)amino)caprylic acid (NAC),
wherein said salt of N-(8-(2-hydroxybenzoyl)amino)caprylic acid (NAC) constitutes at least 90 percent (w/w) of the excipients of the composition.

In further such embodiments, the salt of NAC constitutes at least at least 91 percent (w/w), such as at least 92 percent (w/w), such as at least 93 percent (w/w), such as at least 94 percent (w/w), such as at least 95 percent (w/w) of the excipients of the composition.

In one embodiment, the pharmaceutical composition consists of
a) a PYY compound;
b) excipients, wherein the excipients are
   i. a salt of N-(8-(2-hydroxybenzoyl)amino)caprylic acid (NAC) and
   ii. one or more further excipients and
c) optionally a GLP-1 receptor agonist;
wherein said salt of N-(8-(2-hydroxybenzoyl)amino)caprylic acid (NAC) constitutes at least 90 percent (w/w) of the excipients of the composition.

In further such embodiments, the salt of NAC constitutes at least at least 91 percent (w/w), such as at least 92 percent (w/w), such as at least 93 percent (w/w), such as at least 94 percent (w/w), such as at least 95 percent (w/w) of the excipients of the composition.

In one embodiment, the pharmaceutical composition consists of
a) a PYY compound and
b) a GLP-1 receptor agonist and
c) excipients, wherein the excipients are
   i. a salt of N-(8-(2-hydroxybenzoyl)amino)caprylic acid (NAC) and
   ii. one or more further excipients
wherein said salt of N-(8-(2-hydroxybenzoyl)amino)caprylic acid (NAC) constitutes at least 90 percent (w/w) of the excipients of the composition.

In further such embodiments, the salt of NAC constitutes at least 91 percent (w/w), such as at least 92 percent (w/w), such as at least 93 percent (w/w), such as at least 94 percent (w/w), such as at least 95 percent (w/w) of the excipients of the composition.

In one embodiment, the pharmaceutical composition comprises
a) a PYY compound and
b) a salt of N-(8-(2-hydroxybenzoyl)amino)caprylic acid (NAC),
wherein said salt of N-(8-(2-hydroxybenzoyl)amino)caprylic acid (NAC) constitutes at least 90 percent (w/w), such as at least 95 percent (w/w) of the excipients of the composition.

In one embodiment, the pharmaceutical composition consists of
c) a PYY compound and
d) a salt of N-(8-(2-hydroxybenzoyl)amino)caprylic acid (NAC),
wherein said salt of N-(8-(2-hydroxybenzoyl)amino)caprylic acid (NAC) constitutes at least 90 percent (w/w), such as at least 95 percent (w/w) of the excipients of the composition.

In one embodiment, the pharmaceutical composition consists of
a) a PYY compound and
b) optionally a GLP-1 receptor agonist and
c) excipients, wherein the excipients are
   i. a salt of N-(8-(2-hydroxybenzoyl)amino)caprylic acid (NAC) and
   ii. one or more further excipients and
wherein said salt of N-(8-(2-hydroxybenzoyl)amino)caprylic acid (NAC) constitutes at least 90 percent (w/w), such as at least 95 percent (w/w) of the excipients of the composition.

In further such embodiments, the salt of NAC constitutes above 60 percent (w/w), such as above 70 percent (w/w), such as above 75 percent (w/w), such as 80 percent (w/w) or such as above 90 percent (w/w) of the composition.

In further such embodiments, the salt of NAC constitutes at least 95 percent (w/w), such as at least 96 percent (w/w), such as at least 97 percent (w/w) or such as at least 98 percent (w/w) of the excipients of the composition.

In one embodiment, the pharmaceutical composition consists of
a) a PYY compound and
b) a GLP-1 receptor agonist and
c) excipients, wherein the excipients are
   i. a salt of N-(8-(2-hydroxybenzoyl)amino)caprylic acid (NAC) and
   ii. one or more further excipients
wherein said salt of N-(8-(2-hydroxybenzoyl)amino)caprylic acid (NAC) constitutes at least 90 percent (w/w), such as at least 95 percent (w/w) of the excipients of the composition.

In further such embodiments, the salt of NAC constitutes above 60 percent (w/w), such as above 70 percent (w/w), such as above 75 percent (w/w), such as above 80 percent (w/w) or such as above 90 percent (w/w) of the composition.

In further such embodiments, the salt of NAC constitutes at least 95 percent (w/w), such as at least 96 percent (w/w), such as at least 97 percent (w/w) or such as at least 98 percent (w/w) of the excipients of the composition.

As mentioned above, the content of excipients, besides the delivery agent is according to the invention preferably minimal. In one embodiment, the pharmaceutical composition comprises at least one lubricant.

In one embodiment, the pharmaceutical composition comprises or consists of:
a) a PYY compound,
b) a salt of N-(8-(2-hydroxybenzoyl)amino)caprylic acid (NAC) and
c) at least one lubricant.

In such embodiments, the lubricant may be magnesium stearate or glyceryl dibehenate. In one embodiment, the lubricant is magnesium stearate. In one embodiment, the lubricant is glyceryl dibehenate.

A composition as described above wherein said salt of N-(8-(2-hydroxybenzoyl)amino)caprylic acid (NAC) constitutes at least 90 percent (w/w), such as at least 95 percent (w/w) of the excipients of the composition may further be a composition wherein said salt constitutes at least or above 60 percent (w/w) of the composition.

Likewise the compositions described above wherein said salt constitutes at least or above 60 percent (w/w) of the composition may further be a composition wherein said salt of N-(8-(2-hydroxybenzoyl)amino)caprylic acid (NAC) constitutes at least 90 percent (w/w), such as at least 95 percent (w/w) of the excipients of the composition.

In one embodiment, the pharmaceutical composition comprises or consists of:
a) a PYY compound,
b) a GLP-1 receptor agonist,
c) a salt of N-(8-(2-hydroxybenzoyl)amino)caprylic acid (NAC) and
d) at least one lubricant.

In such embodiments, the lubricant may be magnesium stearate or glyceryl dibehenate. In one embodiment, the lubricant is magnesium stearate. In one embodiment, the lubricant is glyceryl dibehenate.

A composition as described above wherein said salt of N-(8-(2-hydroxybenzoyl)amino)caprylic acid (NAC) constitutes at least 90 percent (w/w), such as at least 95 percent (w/w) of the excipients of the composition may further be a composition wherein said salt constitutes at least or above 60 percent (w/w) of the composition.

Likewise the compositions described above wherein said salt constitutes at least or above 60 percent (w/w) of the composition may further be a composition wherein said salt of N-(8-(2-hydroxybenzoyl)amino)caprylic acid (NAC) constitutes at least 90 percent (w/w), such as at least 95 percent (w/w) of the excipients of the composition.

The pharmaceutical composition may further be a composition wherein the salt of N-(8-(2-hydroxybenzoyl)amino)caprylic acid (NAC) is selected from the group consisting of the sodium salt, potassium salt and/or calcium salt of N-(8-(2-hydroxybenzoyl)amino)caprylic acid (NAC) or alternatively from the group consisting of just the sodium salt and the potassium salt. In one embodiment, the salt of N-(8-(2-hydroxybenzoyl)amino)caprylic acid (NAC) is sodium N-(8-(2-hydroxybenzoyl)amino)caprylate.

In embodiments wherein said salt of N-(8-(2-hydroxybenzoyl)amino)caprylic acid (NAC) constitutes at least 90 percent (w/w) of the excipients of the composition, any further excipients constitutes at most 10 percent (w/w) of the excipients, i.e. any such binder, filler, and/or lubricant/glidant constitutes at the most 10 percent (w/w) of the weight of excipients of the composition. In some embodiments, the excipients of the composition comprise at least or above 90 percent (w/w) delivery agent, and less than 5 percent (w/w) of any further excipients, such as binder, filler, and/or lubricant/glidant. In one embodiment, the excipients of the composition comprise at least 90 percent (w/w) delivery agent and less than 5 percent (w/w) lubricant. In one embodiment, the excipients of the composition comprise at least 90 percent (w/w) delivery agent and less than 3 percent (w/w) lubricant.

In some embodiments, the excipients of the composition comprise at least or above 90 percent (w/w) delivery agent and 0.1-5 percent (w/w), such as 0.5-4 percent (w/w) or 1-3 percent (w/w), of lubricant. In further such embodiments, the excipients of the composition comprise 2-2.5 percent (w/w) of lubricant. In further such embodiments, the excipients of the composition comprise 1-5 percent (w/w) of lubricant. In further such embodiments, the excipients of the composition comprise 1-4 percent (w/w) of lubricant. In further such embodiments, the excipients of the composition comprise 1-3 percent (w/w) of lubricant. In further such embodiments, the excipients of the composition comprise 1-2.5 percent (w/w) of lubricant. In further such embodiments, the excipients of the composition comprise 2-3 percent (w/w) of lubricant. In further such embodiments, the excipients of the composition comprise 2-4 percent (w/w) of lubricant. In further such embodiments, the excipients of the composition comprise 2-5 percent (w/w) of lubricant. In further such embodiments, the excipients of the composition comprise 2.5 percent (w/w) of lubricant.

In embodiments wherein said salt of N-(8-(2-hydroxybenzoyl)amino)caprylic acid (NAC) constitutes at least 95 percent (w/w) of the excipients of the composition, any further excipients of the composition constitute at most 5 percent (w/w) of the excipients, i.e. any such binder, filler, and/or lubricant/glidant constitutes at the most 5 percent (w/w) of the weight of the excipients of the composition. In some embodiments, the excipients of the composition comprise at least 95 percent (w/w) delivery agent and less than 5 percent (w/w) lubricant. In one embodiment, the excipients of the composition comprise at least 95 percent (w/w) delivery agent and less than 3 percent (w/w) lubricant.

In some embodiments, the excipients of the composition comprise at least 95 percent (w/w) delivery agent and 0.1-5 percent (w/w), such as 0.5-4 percent (w/w) or 1-3 percent (w/w), of lubricant. In further such embodiments, the excipients of the composition comprise 2-2.5 percent (w/w) of lubricant.

The pharmaceutical composition according to the invention is preferably produced in a dosage form suitable for oral administration as described herein below. In the following the absolute amounts of the ingredients of the composition of the invention are provided with reference to the content in a dosage unit i.e. per tablet, capsule or sachet.

The pharmaceutical compositions of the invention may in a further embodiment comprise at most 1000 mg of said salt of N-(8-(2-hydroxybenzoyl)amino)caprylic acid per dose unit. In one embodiment, the invention relates to a composition wherein a dose unit comprises at most 500 mg of said salt.

In some embodiments, the amount of the salt of N-(8-(2-hydroxybenzoyl) amino)caprylic acid per dose unit is at least 0.05 mmol, such as at least 0.075 mmol, such as at least 0.1 mmol, such as at least 0.125 mmol, such as at least 0.15 mmol, such as at least 0.20 mmol, at least 0.25 mmol, at least 0.30 mmol, at least 0.35 mmol, at least 0.40 mmol, at least 0.45 mmol, at least 0.50 mmol, at least 0.55 mmol or at least 0.60 mmol.

In some embodiments, the amount of the salt of N-(8-(2-hydroxybenzoyl)amino)caprylic acid per dosage unit of the composition is up to 3 mmol, such as up to 2.75 mmol, such as up to 2.5 mmol, such as up to 2.25 mmol, such as 2 mmol, such as up to 1.5 mmol, up to 1 mmol, up to 0.75 mmol, up to 0.6 mmol, up to 0.5 mmol, up to 0.4 mmol, up to 0.3 mmol and up to 0.2 mmol.

In some embodiments, the amount of the salt of N-(8-(2-hydroxybenzoyl) amino)caprylic acid per dose unit of the composition is in the range of 0.05-3 mmol, 0.10- 2.5 mmol, 0.15- 2.0 mmol, 0.20 - 1.5 mmol, 0.25-1.0 mmol, 0.30-0.75 mmol or such as 0.45-0.65 mmol.

In some embodiments, where the salt of NAC is SNAC, the amount of SNAC in the composition is at least 15 mg, such as at least 20 mg, such as at least 25 mg, such as at least 50 mg, such as at least 75 mg, at least 100 mg, at least 125 mg, at least 150 mg, at least 175 mg, at least 200 mg, at least 225 mg, at least 250 mg, at least 275 mg and at least 300 mg per dose unit.

In some embodiments, where the salt of NAC is SNAC, the amount of SNAC in the composition is up to 1000 mg, such as up to 800 mg, such as up to 600 mg, such as up to 575 mg, such as up to 550 mg, up to 525 mg, up to 500 mg, up to 475 mg, up to 450 mg, up to 425 mg, up to 400 mg, up to 375 mg, up to 350 mg, up to 325 mg per dose unit, or up to 300 mg per dose unit.

In some embodiments, where the salt of NAC is SNAC, the amount of SNAC in the composition is in the range of 15-1000 mg, such as 20-800 mg, such as 25-600 mg, such as 50-500 mg, such as 50-400 mg, such as 75-400 mg, such as 80-350 mg or such as from around 100 to around 300 mg per dose unit.

In one embodiment, where the salt of NAC is SNAC, the amount of SNAC is in the range of 20-200 mg, such as 25-175 mg, such as 75-150 mg, such as 80-120 mg such as around 100 mg per dose unit.

In one embodiment, where the salt of NAC is SNAC, the amount of SNAC is in the range of 200-800 mg, such as 250-400 mg, such as 250-350 mg, such as 275-325 mg, such as around 300 mg per dose unit.

In an embodiment, a dose unit of the pharmaceutical compositions of the invention comprises 0.1-100 mg, 0.2 to 100 mg or 0.5-50 mg of the PYY compound.

In some embodiments, a dose unit of the composition comprises an amount of PYY compound is in the range of 0.2 to 50 mg or 1 to 40 mg.

In some embodiments, a dose unit comprises 0.5-5 mg of the PYY compound, such as 0.75- 4.5 mg, such as 1, 1.5, 2, 2.5 or 3 mg or 3.5, 4, 4.5 mg, such as 1-3 or 3-5 mg of the PYY compound per dose unit.

In some embodiments, a dose unit comprises 2 to 20 mg of the PYY compound, such as 2-15 mg, such as 2, 3, 4, 5, 6 or 7 mg, such as 2, 3, 4 or 5 mg, or such as 8, 10, 12 or 14 mg, such as 15 mg or such as 20 mg of the PYY compound per dose unit.

In some embodiments, a dose unit comprises 5 to 50 mg of the PYY compound, such as 10-45 mg, such as 20, 30 or 40 mg, or such as 25, 35, or 45 mg, or such as 30-50 mg or such as 20-40 mg of the PYY compound per dose unit.

The amount of PYY compound may be varied depending on identity of the PYY compound, the effect desired and indication.

In some embodiments, the pharmaceutical composition comprises a GLP-1 receptor agonist in addition to the PYY compound. In some embodiments, a dose unit of the pharmaceutical compositions of the invention comprises 0.1-100 mg or 0.2 to 100 mg of the GLP-1 receptor agonist. In some embodiments, a dose unit of the composition comprises an amount of the GLP-1 receptor agonist in the range of 0.2 to 50 mg or 1 to 40 mg. In some embodiments, a dose unit comprises 0.5-5 mg of the GLP-1 receptor agonist, such as 0.75-4.5 mg, such as 1, 1.5, 2, 2.5 or 3 mg or 3.5, 4, 4.5 mg, such as 1-3 or 3-5 mg of the GLP-1 receptor agonist per dose unit. In some embodiments, a dose unit comprises 2 to 20 mg of the GLP-1 receptor agonist, such as 2-15 mg, such as 2, 3, 4, 5, 6 or 7 mg, such as 2, 3, 4 or 5 mg, or such as 8, 10, 12 or 14 mg, such as 15 mg or such as 20 mg of the GLP-1 receptor agonist per dose unit. In some embodiments, a dose unit comprises 5 to 50 mg of the GLP-1 receptor agonist, such as 10-45 mg, such as 20, 30 or 40 mg, or such as 25, 35, or 45 mg, or such as 30-50 mg or such as 20-40 mg of the GLP-1 receptor agonist per dose unit. The amount of the GLP-1 receptor agonist may be varied depending on identity of the GLP-1 receptor agonist, the effect desired and the indication, e.g. a higher content may be relevant for treating obesity compared to diabetes.

In a preferred embodiment, a unit dose of the composition comprises 0.5-25 mg magnesium stearate, such as 1-10 mg, such as 2-8 mg or such as 2-5 mg magnesium stearate.

In a preferred embodiment, the amount of magnesium stearate is determined relative to the amount of the salt of NAC, such as SNAC, such that a unit dose of the composition comprises 1-8 mg magnesium stearate or such as 2-5 mg magnesium stearate or 2-3 mg magnesium stearate per 100 mg salt of N-(8-(2-hydroxybenzoyl)amino)caprylic acid, such as SNAC.

In a preferred embodiment, a unit dose of the composition comprises 80-120 mg SNAC, 0.5-5 mg PYY compound and 2-3 mg lubricant.

In a preferred embodiment, a unit dose of the composition comprises 80-120 mg SNAC, 1.5-10 mg PYY compound and 2-3 mg lubricant.

In a preferred embodiment, a unit dose of the composition comprises 80-120 mg SNAC, 5-50 mg PYY compound and 2-3 mg lubricant.

In a preferred embodiment, a unit dose of the composition comprises 250-350 mg SNAC, 0.5-5 mg compound and 3-10 mg lubricant.

In a preferred embodiment, a unit dose of the composition comprises 250-350 mg SNAC, 1.5-10 mg PYY compound and 3-10 mg lubricant.

In a preferred embodiment, a unit dose of the composition comprises 250-350 mg SNAC, 5-50 mg PYY compound and 3-10 mg lubricant.

In a preferred embodiment, a unit dose of the composition comprises 80-120 mg SNAC, 0.5-5 mg PYY compound, 0.5-5 mg GLP- receptor agonist and 2-3 mg lubricant.

In a preferred embodiment, a unit dose of the composition comprises 80-120 mg SNAC, 1.5-10 mg PYY compound, 1.5-10 mg GLP- receptor agonist and 2-3 mg lubricant.

In a preferred embodiment, a unit dose of the composition comprises 80-120 mg SNAC, 5-50 mg PYY compound, 5-50 mg GLP- receptor agonist and 2-3 mg lubricant.

In a preferred embodiment, a unit dose of the composition comprises 250-350 mg SNAC, 0.5-5 mg compound, 0.5-5 mg GLP- receptor agonist and 3-10 mg lubricant.

In a preferred embodiment, a unit dose of the composition comprises 250-350 mg SNAC, 1.5-10 mg PYY compound, 1.5-10 mg GLP- receptor agonist and 3-10 mg lubricant.

In a preferred embodiment, a unit dose of the composition comprises 250-350 mg SNAC, 5-50 mg PYY compound, 5-50 mg GLP- receptor agonist and 3-10 mg lubricant.

In one embodiment, the pharmaceutical composition of the invention has a fast disintegration or dissolution *in vitro.* Disintegration or dissolution may be tested as known in the art and as described herein in Assay I or Assay II.

Dissolution or release may be expressed as the amount of the PYY compound measured in solution after a given period relative to the total content of the PYY compound of the composition. The relative amount may be given in percentage. In one embodiment, the release of the PYY compound from the pharmaceutical composition of the invention is at least 85 % within 15 minutes or at least 95 % within 30 minutes. In one such embodiment, the release is measured at pH 6.8.

In one embodiment, the pharmaceutical composition comprises
a) a PYY compound and
b) a salt of N-(8-(2-hydroxybenzoyl)amino)caprylic acid,
wherein the release of the PYY compound reaches 85 % within 15 minutes or 95 % within 30 minutes. In one embodiment, the release is measured at pH 6.8.

Experiments have demonstrated that the composition of the invention comprising PYY compound/SNAC behave like semaglutide/SNAC compositions with regards to disintegration and dissolution (Examples 2 and 3 herein). In addition, the composition of the invention is bioavailable *in vivo* from oral administration (Example 4 herein). The improved plasma exposure of a PYY compound using a composition according to the invention compared to a PYY compound/SNAC composition prepared according to WO 2012/080471 and WO 2013/139694 can be demonstrated using Assay IV herein, similar to what has previously been observed for semaglutide and other GLP-1 receptor agonists (PCT/EP2019/052487).

In one embodiment, the pharmaceutical composition of the invention provides an early exposure *in vivo.* In one embodiment, the pharmaceutical composition of the invention provides increased exposure of the PYY compound *in vivo.* In one embodiment, the pharmaceutical composition of the invention provides an increased early exposure *in vivo.* Such *in vivo* exposure may be tested in a relevant model, such as the Assay IV described herein. The exposure may also be measured over a predetermined time period and the accumulative dose corrected exposure (AUC) calculated, such as for t=0-30 minutes.

In one embodiment, the invention relates to a pharmaceutical composition wherein the dose corrected exposure at t=30 min is increased relative to PYY compound composition prepared as described in WO2013/139694 substituting the GLP-1 agonist with a PYY compound. Alternatively, the reference may be test composition 1 as described herein.

In one embodiment, the pharmaceutical composition comprises
a) a PYY compound and
b) a salt of N-(8-(2-hydroxybenzoyl)amino)caprylic acid,
wherein the dose corrected exposure at t=30 min is increased relative to a PYY compound composition prepared as type F of WO2013/139694.

In one embodiment, the pharmaceutical composition comprises
a) a PYY compound and
b) a salt of N-(8-(2-hydroxybenzoyl)amino)caprylic acid,
wherein the dose corrected exposure (AUC) for t=0-30 min is increased relative to a PYY compound composition prepared as type F of WO2013/139694.

In one embodiment, the dose corrected exposure (AUC) for t=0-30 min is increased at least 1.2 fold, such as 1.5 fold, such as 2 fold compared to a PYY compound composition prepared as type F of WO2013/139694.

In one embodiment, the pharmaceutical composition comprises
c) a PYY compound and
d) a salt of N-(8-(2-hydroxybenzoyl)amino)caprylic acid,
wherein the dose corrected exposure at t=30 min is increased relative to test composition 1 described herein.

In one embodiment, the pharmaceutical composition comprises
c) a PYY compound and
d) a salt of N-(8-(2-hydroxybenzoyl)amino)caprylic acid,
wherein the dose corrected exposure (AUC) for t=0-30 min is increased relative to test composition 1 described herein.

In one embodiment, the dose corrected exposure (AUC) for t=0-30 min is increased at least 1.2 fold, such as 1.5 fold, such as 2 fold compared to test composition 1 described herein.

### Dosage form

The composition may be administered in several dosage forms, for example as a tablet; a coated tablet; a sachet or a capsule such as hard- or softshell gelatine capsules and all such compositions are considered solid oral dosage forms.

The composition may further be compounded in a drug carrier or drug delivery system, e.g. in order to improve stability and/or solubility or further improve bioavailability. The composition may be a freeze-dried or spray-dried composition.

The composition may be in the form of a dose unit, such as a tablet. In some embodiments, the weight of the unit dose is in the range of 50 mg to 1000 mg, such as in the range of 50-750 mg, or such as in the range of 100-500 mg. In some embodiments, the weight of the dose unit is in the range of 75 mg to 350 mg, such as in the range of 100-300 mg or such as in the range of 200-350 mg. In some embodiments, the weight of the dose unit is in the range of 100 mg to 400 mg, such as in the range of 50-300 mg or such as in the range of 200-400 mg.

In some embodiments, the composition may be granulated prior to being compacted and e.g. compressed into tablets. The composition may comprise an intragranular part and/or an extragranular part, wherein the intragranular part has been granulated and the extragranular part has been added after granulation. In some embodiments, the composition may comprise one or more intragranular parts and/or an extragranular part, wherein the one or more intragranular parts have been granulated and the extragranular part has been added after granulation.

The intragranular part may comprise the PYY compound, the delivery agent and/or an excipient, such as a lubricant and/or glidant. In some embodiments, the intragranular part comprises the delivery agent and a lubricant and/or a glidant and optionally the GLP-1 receptor agonist. In some embodiments, the composition may comprise both a PYY compound and a GLP-1 receptor agonist. In one such embodiment, said composition may be made from one intragranular part comprising the PYY compound, the GLP-1 receptor agonist, the delivery agent and a lubricant and/or a glidant. Also or alternatively, in one embodiment, said composition may be made by mixing two intragranular parts: i) one intragranular part comprising the PYY compound, the delivery agent and a lubricant and/or glidant and ii) another intragranular part comprising the GLP-1 receptor agonist, the delivery agent and a lubricant and/or glidant.

In some embodiments, the extragranular part comprises the PYY compound, and/or a lubricant and/or a glidant, such as magnesium stearate and optionally the GLP-1 receptor agonist. In some embodiments, the extragranular part comprises the PYY compound and optionally the GLP-1 receptor agonist. In some embodiments, the extragranular part comprises an excipient, such as a lubricant and/or glidant, such as magnesium stearate.

In further embodiments, the intragranular part comprises the PYY compound, the delivery agent and the lubricant and/or a glidant. In such embodiments, the granulate may be directly compressed into tablets and the tablets have no extragranular part.

### Preparation of composition

Preparation of a composition according to the invention may be performed according to methods known in the art.

To prepare a dry **blend** of tabletting material, the various components are optionally delumped or sieved, weighed and then combined. The mixing of the components may be carried out until a homogeneous blend is obtained.

The terms "granulate" and "granules" are used interchangeably herein to refer to particles of composition material which may be prepared as described above. The term refers broadly to pharmaceutical ingredients in the form of particles, granules, and aggregates which are used in the preparation of solid dose formulations. Generally, granules are obtained by processing a powder or a blend to obtain a solid which is subsequently broken down to obtain granules of the desired size.

If granules are to be used in the tabletting material, granules may be produced in a manner known to a person skilled in the art, for example using **wet granulation** methods known for the production of "built-up" granules or "broken-down" granules. Methods for the formation of **built-up granules** may operate continuously and comprise, for example simultaneously spraying the granulation mass with granulation solution and drying, for example in a drum granulator, in pan granulators, on disc granulators, in a fluidized bed, by spray-drying, spray-granulation or spray-solidifying, or operate discontinuously, for example in a fluidized bed, in a rotary fluid bed, in a batch mixer, such as a high shear mixer or a low shear mixer, or in a spray-drying drum. Methods for the production of **broken-down granules,** which may be carried out discontinuously and in which the granulation mass first forms a wet aggregate with the granulation solution, which is subsequently comminuted or by other means formed into granules of the desired size and the granules may then be dried. Suitable equipment for the wet granulation step are planetary mixers, low shear mixers, high shear mixers, extruders and spheronizers, such as an apparatus from the companies Loedige, Glatt, Diosna, Fielder, Collette, Aeschbach, Alexanderwerk, Ytron, Wyss & Probst, Werner & Pfleiderer, HKD, Loser, Fuji, Nica, Caleva and Gabler. Granules may also be formed by **dry granulation** techniques in which one or more of the excipient(s) and/or the active pharmaceutical ingredient is compressed to form relatively large moldings, for example slugs or ribbons, which are comminuted by grinding, and the ground material serves as the tabletting material to be later compacted. Suitable equipment for dry granulation is, but not limited to, roller compaction equipment from Gerteis such as Gerteis MICRO-PACTOR, MINI-PACTOR and MACRO-PACTOR.

To **compact** the tabletting material into a solid oral dosage form, for example a tablet, a tablet press may be used. In a tablet press, the tabletting material is filled (e.g. force fed or gravity fed) into a die cavity. The tabletting material is then compacted by a set of punches applying pressure. Subsequently, the resulting compact, or tablet is ejected from the tablet press. The above mentioned tabletting process is subsequently referred to herein as the "compaction process". Suitable **tablet presses** include, but are not limited to, rotary tablet presses and eccentric tablet presses. Examples of tablet presses include, but are not limited to, the Fette 102i (Fette GmbH), the Korsch XL100, the Korsch PH 106 rotary tablet press (Korsch AG, Germany), the Korsch EK-O eccentric tabletting press (Korsch AG, Germany), the Romaco Kilian STYL'ONE Evo tablet press and the Manesty F-Press (Manesty Machines Ltd., United Kingdom).

In general, granulates may be prepared by wet, melt or dry granulation, preferably dry granulation. Granules of i, ii and/or iii and/or optionally iv. may thus be obtained by dry granulation of a blend hereof, such as by roller compaction. Also or alternatively, in one embodiment, wet granulation may be used to obtain the granules. This material can then be used directly or further refined to obtain the final granules.

In one embodiment, the composition comprises at least one granulate. In one embodiment, the composition comprises one type of granulate. The composition may alternatively comprise two types of granulates.

In one embodiment, the invention relates to a composition comprising
i. a PYY compound,
ii. a salt of N-(8-(2-hydroxybenzoyl)amino)caprylic acid (NAC) and
iii. a lubricant
wherein the composition comprises a granulate of ii. and optionally iii. In a further embodiment, the granular part may comprise i. and ii. and optionally iii.

In one embodiment, the invention relates to a composition comprising
i. a PYY compound,
ii. a salt of N-(8-(2-hydroxybenzoyl)amino)caprylic acid (NAC),
iii. a lubricant and
iv. a GLP-1 receptor agonist
wherein the composition comprises a granulate of ii. and optionally iii. In a further embodiment, the granular part may comprise i., ii., and iv. and optionally iii.

In one embodiment, the invention relates to a composition comprising
i. a PYY compound,
ii. a salt of N-(8-(2-hydroxybenzoyl)amino)caprylic acid (NAC),
iii. a lubricant and
iv. a GLP-1 receptor agonist
wherein the composition comprises two granulates: a first granulate of i. and ii. and optionally iii. and a second granulate of iv., ii., and optionally iii.

The granulation may be obtained by various methods as described above, wherein i, ii and/or iii and/or optionally iv. are initially mixed either as powders or by the preparation of a solution comprising both ingredients.

In some embodiments, the method of preparation of the tablet comprises; a) granulating a mixture comprising the delivery agent and optionally a lubricant; b) blending the granulate of a) with a PYY compound and optionally additional lubricant, and then c) compressing the blend of b) into tablets.

In one embodiment, the method of preparation of the tablet comprises; a) granulating a mixture comprising the delivery agent and optionally a lubricant; b) blending the granulate of a) with a PYY compound and a GLP-1 receptor agonist and optionally additional lubricant, and then c) compressing the blend of b) into tablets.

In some embodiments, the method of preparation of the tablet comprises; a) granulating a mixture comprising the delivery agent, the PYY compound and optionally a lubricant and b) compressing the granulate of a) into tablets and optionally including additional lubricant. In some embodiments, the method of preparation of the tablet comprises; a) granulating a mixture comprising the delivery agent, the PYY compound, the GLP-1 receptor agonist and optionally a lubricant and b) compressing the granulate of a) into tablets and optionally including additional lubricant. In some embodiments, the method of preparation of the tablet comprises; a) granulating a first a mixture comprising the delivery agent, the PYY compound, and optionally a lubricant, b) granulating a second mixture comprising the delivery agent, the GLP-1 receptor agonist, and optionally a lubricant and c) mixing the granules of a) and b) and compressing the mixture of granulates of a) and b) into tablets and optionally including additional lubricant.

To obtain a homogenous granulate one or more sieving step(s) can be included prior to the final dry granulation step/roller compaction or tablet compression step.

Finally, additional excipient(s), such as a lubricant may be added prior to tablet compression forming an extragranular part.

### Pharmaceutical Indications

The present invention also relates to a composition of the invention for use as a medicament. In some embodiment, the composition of the invention may be used for the following medical treatments, all preferably relating one way or another to diabetes and/or obesity:
(i) prevention and/or treatment of all forms of diabetes, such as hyperglycemia, type 2 diabetes, impaired glucose tolerance, type 1 diabetes, non-insulin dependent diabetes, MODY (maturity onset diabetes of the young), gestational diabetes, and/or for reduction of HbA1C;
(ii) delaying or preventing diabetic disease progression, such as progression in type 2 diabetes, delaying the progression of impaired glucose tolerance (IGT) to insulin requiring type 2 diabetes, delaying or preventing insulin resistance, and/or delaying the progression of non-insulin requiring type 2 diabetes to insulin requiring type 2 diabetes;
(iii) improving β-cell function, such as decreasing β-cell apoptosis, increasing β-cell function and/or β-cell mass, and/or for restoring glucose sensitivity to β-cells;
(iv) prevention and/or treatment of eating disorders, such as obesity, e.g. by decreasing food intake, reducing body weight, suppressing appetite, inducing satiety; treating or preventing binge eating disorder, bulimia nervosa, and/or obesity induced by administration of an antipsychotic or a steroid; reduction of gastric motility; delaying gastric emptying; increasing physical mobility; and/or prevention and/or treatment of comorbidities to obesity, such as osteoarthritis and/or urine incontinence;
(v) prevention and/or treatment of diabetic complications, such as angiopathy; neuropathy, including peripheral neuropathy; nephropathy; and/or retinopathy;
(vi) improving lipid parameters, such as prevention and/or treatment of dyslipidemia, lowering total serum lipids; increasing HDL; lowering small, dense LDL; lowering VLDL; lowering triglycerides; lowering cholesterol; lowering plasma levels of lipoprotein a (Lp(a)) in a human; inhibiting generation of apolipoprotein a (apo(a)) in vitro and/or in vivo;
(vii) prevention and/or treatment of cardiovascular diseases;
(viii) prevention and/or treatment of liver disorders, such as hepatic steatosis, non-alcoholic fatty liver disease (NAFLD), non-alcoholic steatohepatitis (NASH), liver inflammation or fatty liver;
(ix) prevention and/or treatment of sleep apnoea; and/or
(x) weight maintenance after successful weight loss (either drug induced or by diet and exercise) - i.e. prevention of weight gain after successful weight loss.

The following indications are particularly preferred: Type 2 diabetes, and/or obesity.

### Method of treatment

The invention further relates to a method of treating a subject in need thereof, comprising administering a therapeutically effective amount of a composition according to the present invention to said subject. In one embodiment, the method of treatment is for treatment of diabetes or obesity and/or the further indications specified above.

In some embodiments, a method for treating obesity is described comprising administering to a subject in need thereof a therapeutically effective amount of a pharmaceutical composition comprising a PYY compound, a salt of N-(8-(2-hydroxybenzoyl)amino)caprylic acid (NAC), and optionally, a lubricant. In some embodiments, a method for treating obesity is described comprising administering to a subject in need thereof a therapeutically effective amount of a pharmaceutical composition comprising a PYY compound, a salt of N-(8-(2-hydroxybenzoyl)amino)caprylic acid (NAC), optionally a lubricant and optionally, a GLP-1 receptor agonist.

In some embodiments, a method for treating obesity is described comprising administering to a subject in need thereof a therapeutically effective amount of a pharmaceutical composition comprising
0.5-100 mg of a PYY compound,
25-600 mg of salt of N-(8-(2-hydroxybenzoyl)amino)caprylic acid (NAC),
1-10 mg lubricant as described herein.

In some embodiments, a method for treating obesity is described comprising administering to a subject in need thereof a therapeutically effective amount of a pharmaceutical composition comprising
0.5-100 mg of a PYY compound,
25-600 mg of salt of N-(8-(2-hydroxybenzoyl)amino)caprylic acid (NAC),
1-10 mg lubricant, and
0.5-50 mg of a GLP-1 receptor agonist as described herein.

In a preferred embodiment, the salt of N-(8-(2-hydroxybenzoyl)amino)caprylic acid constitutes at least 90 percent (w/w) of the excipients of the composition. In a preferred embodiment, the salt of N-(8-(2-hydroxybenzoyl)amino)caprylic acid constitutes at least 95 percent (w/w) of the excipients of the composition.

In an alternative embodiment, a method for treating obesity is described comprising administering to a subject in need thereof a therapeutically effective amount of a pharmaceutical composition comprising about 1-30 mg of a PYY compound, about 100-300 mg of salt of N-(8-(2-hydroxybenzoyl)amino)caprylic acid (NAC), about 2-8 mg of magnesium stearate, and optionally about 1-20 mg of a GLP-1 receptor agonist.

In some embodiments, the PYY compound is selected from compounds 1-63, 65-71 as disclosed herein and in WO2016/198682 (compounds 1-63, 65-71, example 1). In some embodiments, the PYY compound is selected from compound 4 (N{alpha-4}-(3-Methylbutanoyl)-N{Epsilon-7}-6-[[(4S)-4-carboxy-4-(17-carboxy-heptadecanoylamino)butanoyl]amino]hexanoyl-[Arg4, Lys7,Pro9,Gln18,Tyr28,Trp30, Leu31]hPYY(4-36)), compound 20 (N{alpha-4}-(3-Methylbutanoyl)-N{Epsilon-7}-6-[[(4S)-4-carboxy-4-(17-carboxy-heptadecanoylamino)butanoyl]amino]hexanoyl-[Arg4,Lys7,Gln18,Ile24, Tyr28,Trp30,Leu31]hPYY(4-36)), and compound 32 (N{alpha-4}-(3-Methylbutanoyl)-N{Epsilon-7}-6-[[(4S)-4-carboxy-4-(17-carboxy-heptadecanoylamino)butanoyl]amino]hexanoyl-[Arg4,Lys7,Gln18,Ile22,Tyr28, Trp30, Leu31]hPYY(4-36)).

In some embodiments, the GLP-1 receptor agonist is semaglutide having a formula of N-epsilon26-[2-(2-{2-[2-(2-{2-[(S)-4-carboxy-4-(17-carboxy-heptadecanoylamino)butyrylamino]ethoxy}ethoxy)acetylamino]ethoxy}ethoxy)acetyl] [Aib8,Arg34]GLP-1(7-37) and the salt of N-(8-(2-hydroxybenzoyl)amino)caprylic acid (NAC) is sodium N-(8-(2-hydroxybenzoyl)amino)caprylic acid (SNAC). Various examples of a lubricant are described, including magnesium stearate. The composition is administered orally and is in a form of a table, capsule or a sachet.

In a further such embodiments one or more dose units may be administered to said subject in need.

### Embodiments

The invention is further described by the following non-limiting embodiments of the invention:
1. A pharmaceutical composition comprising
   a) a PYY compound and
   b) a salt of N-(8-(2-hydroxybenzoyl)amino)caprylic acid
   wherein said salt of N-(8-(2-hydroxybenzoyl)amino)caprylic acid constitutes at least 60 percent (w/w) of the composition.
2. A pharmaceutical composition comprising
   a) a PYY compound and
   b) a salt of N-(8-(2-hydroxybenzoyl)amino)caprylic acid
   wherein said salt of N-(8-(2-hydroxybenzoyl)amino)caprylic acid constitutes at least 90 percent (w/w), such as at least 95 percent (w/w) of the excipients of the composition.
3. A pharmaceutical composition comprising
   a) a PYY compound and
   b) excipients, wherein the excipients are
      i. a salt of N-(8-(2-hydroxybenzoyl)amino)caprylic acid and
      ii. one or more further excipients
   wherein said salt of N-(8-(2-hydroxybenzoyl)amino)caprylic acid constitutes at least 90 percent (w/w), such as at least 95 percent (w/w) of the excipients of the composition.
4. The pharmaceutical composition according to any one of embodiments 1-3, wherein the composition further comprises at least one lubricant.
5. The pharmaceutical composition according to any one of embodiments 1-4, wherein the composition further comprises a GLP-1 receptor agonist.
6. A pharmaceutical composition comprising:
   a) a PYY compound,
   b) a salt of N-(8-(2-hydroxybenzoyl)amino)caprylic acid and
   c) at least one lubricant.
7. The pharmaceutical composition according to embodiment 6 consisting of:
   a) a PYY compound,
   b) a GLP-1 receptor agonist,
   c) a salt of N-(8-(2-hydroxybenzoyl)amino)caprylic acid and
   d) at least one lubricant.
8. The pharmaceutical composition according to any one of embodiments 4-7, wherein the lubricant is magnesium stearate.
9. The pharmaceutical composition according any one of the preceding embodiments, wherein the composition comprises 1-8 mg, such as 2-5 mg or such as 2-3 mg magnesium stearate per 100 mg salt of N-(8-(2-hydroxybenzoyl)amino)caprylic acid.
10. The pharmaceutical composition according to any one of the preceding embodiments, wherein said salt of N-(8-(2-hydroxybenzoyl)amino)caprylic acid constitutes at least 60 percent (w/w) of the composition.
11. The pharmaceutical composition according to any one of the preceding embodiments, wherein said salt of N-(8-(2-hydroxybenzoyl)amino)caprylic acid constitutes at least 95 percent (w/w) of the excipients of the composition.
12. The pharmaceutical composition according to any one of the preceding embodiments, wherein the salt of N-(8-(2-hydroxybenzoyl)amino)caprylic acid is selected from the group consisting of the sodium salt, potassium salt and/or calcium salt of N-(8-(2-hydroxybenzoyl)amino)caprylic acid.
13. The pharmaceutical composition according to any one of the preceding embodiments, wherein the salt of N-(8-(2-hydroxybenzoyl)amino)caprylic acid is sodium N-(8-(2-hydroxybenzoyl)amino)caprylate (SNAC).
14. The pharmaceutical composition according to any one of the preceding embodiments, wherein a dose unit comprises at most 1000 mg of said salt of N-(8-(2-hydroxybenzoyl)amino)caprylic acid.
15. The pharmaceutical composition according to any one of the preceding embodiments, wherein a dose unit comprises 0.5-100 mg of the PYY compound.
16. The pharmaceutical composition according to any one of the preceding embodiments, wherein the PYY compound is a PYY analogue.
17. The pharmaceutical composition according to any one of the preceding embodiments, wherein the PYY compound is a PYY derivative.
18. The composition according to any one of the preceding embodiments, wherein the PYY compound is selected from the compounds 1-63, 65-71 of WO2016/198682.
19. The pharmaceutical composition according to embodiment 17-18, wherein PYY derivative comprises a fatty acid or a fatty diacid.
20. The pharmaceutical composition according to any of embodiments 17-19, wherein the PYY derivative comprises a C16, C18 or C20 fatty acid or a C16, C18 or C20 fatty diacid.
21. The pharmaceutical composition according to any one of the preceding embodiments, wherein the PYY compound is a PYY derivative comprising a modifying group attached to the backbone of the PYY compound, wherein said modifying group is defined by A-**[B]ᵣ-C- or A-[B]ᵣ-C-[B]_{w},** wherein
   A- is selected from Chem. 1 and Chem. 2

      Chem. 1: HOOC-(CH₂)ₚ-CO-*,

      Chem. 2: HO₃S-(CH₂)_{q}-CO-*

      wherein p is an integer in the range of 14-18, and q is an integer in the range of 15-17;
   B- is Chem. 3

      Chem. 3: *[NH-CH(COOH)-(CH₂)₂-CO-]-*,

      r is an integer in the range of 1-3;
      w is an integer in the range of 1-3; and
   C- is absent or selected from Chem. 4 and Chem. 5

      Chem. 4: *[NH-(CH₂)₂-[O-(CH₂)_{2]s}-O-(CH₂)t-CO-]ᵤ-*

      Chem. 5: *[NH-(CH₂)ᵥ-CO-]ₓ-*

      wherein s is an integer in the range of 1-3, t is an integer in the range of 1-3, u is an integer in the range of 1-4, v is an integer in the range of 3-7, and x is an integer in the range of 1-3;
      wherein * denotes the points of attachment, and wherein A, B, and C are interconnected via amide bonds and in the sequence indicated via said point of attachments; or a pharmaceutically acceptable salt, amide, or ester of said PYY derivative; and wherein if the modifying group is A-B-C-B, C cannot be absent.
22. The pharmaceutical composition according to embodiment 21, wherein the modifying group is defined by **A-[B]ᵣ-C-,** wherein
   A- is Chem. 1

      Chem. 1: HOOC-(CH₂)ₚ-CO-*,

      wherein p is 16;
   **B-** is Chem. 3

      Chem. 3: *[NH-CH(COOH)-(CH₂)₂-CO-]-*,

      r is 1; and
   **C-** is Chem. 5

      Chem. 5: *[NH-(CH₂)ᵥ-CO-]ₓ-*

      wherein v is 5 and x is 1.
23. The pharmaceutical composition according to any one of embodiments 21-22, wherein the modifying group is attached to the backbone of the PYY compound at a position corresponding to position 7 or 10 of hPYY(1-36).
24. The composition according to any one of the preceding embodiments, wherein the PYY compound is a PYY derivative having a maximum of 10 amino acid modifications as compared to hPYY(3-36), wherein the PYY derivative comprises
   a) lysine at the position corresponding to position 7 or 10 of hPYY(1-36);
   b) tryptophan at the position corresponding to position 30 of hPYY(1-36);
   c) leucine at the position corresponding to position 31 of hPYY(1-36);
   d) tyrosine at the position corresponding to position 28 of hPYY(1-36) and/or isoleucine at the position corresponding to position 22 of hPYY(1-36); and
   e) a modifying group attached to the epsilon amino group of said lysine at the position corresponding to position 7 or 10 of hPYY(1-36) (SEQ ID NO:1),
      wherein said modifying group is defined by **A-[B]ᵣ-C- or A-[B]ᵣ-C-[B]_{w},** wherein
      **A-** is selected from Chem. 1 and Chem. 2

         Chem. 1: HOOC-(CH₂)ₚ-CO-*,

         Chem. 2: HO₃S-(CH₂)_{q}-CO-*

         wherein p is an integer in the range of 14-18, and q is an integer in the range of 15-17;
      **B- is** Chem. 3

         Chem. 3: *[NH-CH(COOH)-(CH₂)₂-CO-]-*,

         r is an integer in the range of 1-3; w is an integer in the range of 1-3; and
      **C-** is absent or selected from Chem. 4 and Chem. 5

         Chem. 4: *[NH-(CH₂)₂-[O-(CH₂)₂]ₛ-O-(CH₂)ₜ-CO-]ᵤ-*

         Chem. 5: *[NH-(CH₂)ᵥ-CO-]ₓ-*

         wherein s is an integer in the range of 1-3, t is an integer in the range of 1-3, u is an integer in the range of 1-4, v is an integer in the range of 3-7, and x is an integer in the range of 1-3;
   wherein * denotes the points of attachment, and wherein A, B, and C are interconnected via amide bonds and in the sequence indicated via said point of attachments; or a pharmaceutically acceptable salt, amide, or ester of said PYY derivative; and wherein if the modifying group is A-B-C-B, C cannot be absent.
25. The composition according to any one of the preceding embodiments, wherein the compound is a PYY derivative having a maximum of 10 amino acid modifications as compared to hPYY(3-36), wherein the PYY derivative comprises
   a) arginine at the position corresponding to position 4 of hPYY(1-36);
   b) lysine at the position corresponding to position 7 of hPYY(1-36);
   c) Glutamine at the position corresponding to position 18 of hPYY(1-36);
   d) tryptophan at the position corresponding to position 30 of hPYY(1-36);
   e) leucine at the position corresponding to position 31 of hPYY(1-36);
   f) tyrosine at the position corresponding to position 28 of hPYY(1-36); and
   g) a modifying group attached to the epsilon amino group of said lysine at the position corresponding to position 7 of hPYY(1-36) (SEQ ID NO:1),
      wherein said modifying group is defined by **A-B-C-,** wherein
      **A-** is Chem. 1

         Chem. 1: HOOC-(CH₂)ₚ-CO-*,

         wherein p is an integer in the range of 14-18;
      **B-** is Chem. 3

         Chem. 3: *[NH-CH(COOH)-(CH₂)₂-CO-]-*; and
      **C-** is Chem. 5

         Chem. 5: *[NH-(CH₂)ᵥ-CO-]ₓ-*

         wherein v is 5, and x is an integer in the range of 1;
   wherein * denotes the points of attachment, and wherein A, B, and C are interconnected via amide bonds and in the sequence indicated via said point of attachments; or a pharmaceutically acceptable salt, amide, or ester thereof.
26. The pharmaceutical composition according to any one of the preceding embodiments, wherein the PYY compound is selected from the group consisting of:
   N{Epsilon-7}-[(4S)-4-carboxy-4-[[(4S)-4-carboxy-4-(17-carboxyheptadecanoylamino)-butanoyl]amino]butanoyl]-[Arg4,Lys7,Gln18,Tyr28,Trp30,Leu31]hPYY(3-36) (Compound 1, SEQ ID NO: 3);
   N{alpha-4}-(3-Methylbutanoyl)-N{Epsilon-7}-[(4S)-4-carboxy-4-(17-carboxy-heptadecanoylamino)butanoyl]-[Arg4,Lys7,Gln18,Tyr28,Trp30,Leu31]hPYY(4-36) (Compound 2, SEQ ID NO: 4);
   N{alpha-4}-(3-Methylbutanoyl)-N{Epsilon-7}-[2-[2-[2-[[2-[2-[2-[[(4S)-4-carboxy-4-(17-carboxyheptadecanoylamino)butanoyl]amino]ethoxy]ethoxy]acetyl]amino]-ethoxy]ethoxy]acetyl]-[Arg4,Lys7,Gln18,Tyr28,Trp30,Leu31]hPYY(4-36) (Compound 3, SEQ ID NO: 4);
   N{alpha-4}-(3-Methylbutanoyl)-N{Epsilon-7}-6-[[(4S)-4-carboxy-4-(17-carboxy-heptadecanoylamino)butanoyl]amino]hexanoyl-[Arg4, Lys7, Pro9,Gln 18,Tyr28, Trp30,Leu31]hPYY(4-36) (Compound 4, SEQ ID NO: 5);
   N{alpha-4}-(3-Methylbutanoyl)-N{Epsilon-7}-[2-[2-[2-[[2-[2-[2-[[(4S)-4-carboxy-4-[[(4S)-4-carboxy-4-(17-carboxyheptadecanoylamino)butanoyl]amino]butanoyl]amino]-ethoxy]ethoxy]-acetyl]amino]ethoxy]ethoxy]acetyl]-[Arg4,Lys7,Gln18,Tyr28,Trp30, Leu31]hPYY(4-36) (Compound 5, SEQ ID NO: 4);
   N{alpha-4}-(3-Methylbutanoyl)-N{Epsilon-7}-[(4S)-4-carboxy-4-(15-carboxy-pentadecanoylamino)butanoyl]-[Arg4,Lys7,Gln18,Tyr28,Trp30,Leu31]hPYY(4-36) (Compound 6, SEQ ID NO: 4);
   N{alpha-4}-(3-Methylbutanoyl)-N{Epsilon-7}-[(4S)-4-carboxy-4-(19-carboxy-nonadecanoylamino)butanoyl]-[Arg4,Lys7,GIn18,Tyr28,Trp30,Leu31]hPYY(4-36) (Compound 7, SEQ ID NO: 4);
   N{alpha-4}-(3-Methyl butanoyl)-N{Epsilon-7}-[2-[2-[2-[[(4S)-4-carboxy-4-(17-carboxy-heptadecanoylamino)butanoyl]amino]ethoxy]ethoxy]acetyl]-[Arg4,Lys7,GIn18,Tyr28, Trp30,Leu31]hPYY(4-36) (Compound 8, SEQ ID NO: 4);
   N{alpha-4}-(3-Methylbutanoyl)-N{Epsilon-7}-[(4S)-4-carboxy-4-(16-sulfohexadecanoyl-amino)butanoyl]-[Arg4,Lys7,Gln18,Tyr28,Trp30,Leu31]hPYY(4-36) (Compound 9, SEQ ID NO: 4);
   N{alpha-4}-(3-Methyl butanoyl)-N{Epsilon-7}-6-[[(4S)-4-carboxy-4-(19-carboxy-nonadecanoylamino)butanoyl]amino]hexanoyl-[Arg4,Lys7,Gln18,Tyr28,Trp30, Leu31]hPYY(4-36) (Compound 10, SEQ ID NO: 4);
   N{alpha-4}-(3-Methylbutanoyl)-N{Epsilon-7}-[(4S)-4-carboxy-4-[[(4S)-4-carboxy-4-(17-carboxyheptadecanoylamino)butanoyl]amino]butanoyl]-[Arg4,Lys7,Pro9,Gln18, Tyr28,Trp30,Leu31]hPYY(4-36) (Compound 11, SEQ ID NO: 5);
   N{alpha-4}-(3-Methylbutanoyl)-N{Epsilon-7}-[(4S)-4-carboxy-4-[[(4S)-4-carboxy-4-(19-carboxynonadecanoylamino)butanoyl]amino]butanoyl]-[Arg4,Lys7,Pro9,Gln18, Tyr28,Trp30,Leu31]hPYY(4-36) (Compound 12, SEQ ID NO: 5);
   N{alpha-4}-(3-Methylbutanoyl)-N{Epsilon-7}-[2-[2-[2-[[2-[2-[2-[[(4S)-4-carboxy-4-(17-carboxyheptadecanoylamino)butanoyl]amino]ethoxy]ethoxy]acetyl]amino]-ethoxy]ethoxy]acetyl]-[Arg4,Lys7,Ser9,Gln18,Tyr28,Trp30,Leu31]hPYY(4-36) (Compound 13, SEQ ID NO: 6);
   N{alpha-4}-(3-Methylbutanoyl)-N{Epsilon-7}-[2-[2-[2-[[2-[2-[2-[[(4S)-4-carboxy-4-(17-carboxyheptadecanoylamino)butanoyl]amino]ethoxy]ethoxy]acetyl]amino]-ethoxy]ethoxy]acetyl]-[Arg4,Lys7,Thr9,Gln18,Tyr28,Trp30,Leu31]hPYY(4-36) (Compound 14, SEQ ID NO: 7);
   N{alpha-4}-(3-Methylbutanoyl)-N{Epsilon-7}-[(4S)-4-carboxy-4-(17-carboxy-heptadecanoylamino)butanoyl]-[Arg4,Lys7,Thr13,Gln18,Tyr28,Trp30,Leu31]hPYY(4-36) (Compound 15, SEQ ID NO: 8);
   N{alpha-4}-(3-Methylbutanoyl)-N{Epsilon-7}-[2-[2-[2-[[2-[2-[2-[[(4S)-4-carboxy-4-(17-carboxyheptadecanoylamino)butanoyl]amino]ethoxy]ethoxy]acetyl]amino]-ethoxy]ethoxy]acetyl]-[Arg4, Lys7,Thr13,Gln18,Tyr28,Trp30, Leu31]hPYY(4-36) (Compound 16, SEQ ID NO: 8);
   N{alpha-4}-(3-Methylbutanoyl)-N{Epsilon-7}-[(4S)-4-carboxy-4-(17-carboxy-heptadecanoylamino)butanoyl]-[Arg4,Lys7,GIn18,Ala24,Tyr28,Trp30,Leu31]hPYY(4-36) (Compound 17, SEQ ID NO: 9);
   N{alpha-4}-(3-Methylbutanoyl)-N{Epsilon-7}-[(4S)-4-carboxy-4-(17-carboxy-heptadecanoylamino)butanoyl]-[Arg4,Lys7,Gln18,lIe24,Tyr28,Trp30,Leu31]hPYY(4-36) (Compound 18, SEQ ID NO: 10);
   N{alpha-4}-(3-Methylbutanoyl)-N{Epsilon-7}-6-[[(4S)-4-carboxy-4-(19-carboxy-nonadecanoylamino)butanoyl]amino]hexanoyl-[Arg4,Lys7,Gln18,Ile24,Tyr28,Trp30, Leu31]hPYY(4-36) (Compound 19, SEQ ID NO: 10);
   N{alpha-4}-(3-Methylbutanoyl)-N{Epsilon-7}-6-[[(4S)-4-carboxy-4-(17-carboxy-heptadecanoylamino)butanoyl]amino]hexanoyl-[Arg4,Lys7,Gln18,Ile24, Tyr28,Trp30,Leu31]hPYY(4-36) (Compound 20, SEQ ID NO: 10);
   N{alpha-4}-}-(3-Methylbutanoyl)-N{Epsilon-7}-[2-[2-[2-[[2-[2-[2-[[(4S)-4-carboxy-4-(17-carboxy-heptadecanoylamino)butanoyl]amino]ethoxy]ethoxy]acetyl]amino]ethoxy]ethoxy]acetyl]-[Arg4,Lys7,Pro9,Gln18,Tyr28,Trp30,Leu31]hPYY(4-36) (Compound 21, SEQ ID NO: 5);
   N{Alpha-4}-acetyl,N{Epsilon-7}-[(4S)-4-carboxy-4-(17-carboxyheptadecanoylamino)-butanoyl]-[Arg4,Lys7,Gln18,lle24,Tyr28,Trp30,Leu31]hPYY(4-36) (Compound 22, SEQ ID NO: 11);
   N{Epsilon-71-[(4S)-4-carboxy-4-[[(4S)-4-carboxy-4-(17-carboxyheptadecanoylamino)-butanoyl]amino]butanoyl]-[Arg4,Lys7,Gln18,lle22,Trp30,Leu31]hPYY(3-36) (Compound 23, SEQ ID NO: 12);
   N{Epsilon-7}-[2-[2-[2-[[2-[2-[2-[[(4S)-4-carboxy-4-(17-carboxyheptadecanoylamino)-butanoyl]amino]ethoxy]ethoxy]acetyl]amino]ethoxy]ethoxy]acetyl]-[Arg4,Lys7,Gln18, Ile22,Trp30,Leu31]hPYY(3-36) (Compound 24, SEQ ID NO: 12);
   N{alpha-4}-(3-Methylbutanoyl)-N{Epsilon-7}-[(4S)-4-carboxy-4-(17-carboxy-heptadecanoylamino)butanoyl]-[Arg4,Lys7,Gln18,Ile22,Trp30,Leu31]hPYY(4-36) (Compound 25, SEQ ID NO: 13);
   N{alpha-4}-(3-Methylbutanoyl)-N{Epsilon-7}-[2-[2-[2-[[2-[2-[2-[[(4S)-4-carboxy-4-(17-carboxyheptadecanoylamino)butanoyl]amino]ethoxy]ethoxy]acetyl]amino]-ethoxy]ethoxy]acetyl]-[Arg4,Lys7,Gln18,lle22,Trp30,Leu31]hPYY(4-36) (Compound 26, SEQ ID NO: 13);
   N{alpha-4}-(3-Methylbutanoyl)-N{Epsilon-7}-[(4S)-4-carboxy-4-(17-carboxy-heptadecanoylamino)butanoyl]-[Arg4,Lys7,Pro9,Gln18,Ile22,Trp30,Leu31]hPYY(4-36) (Compound 27, SEQ ID NO: 14);
   N{alpha-4}-(3-Methylbutanoyl)-N{Epsilon-7}-[2-[2-[2-[[2-[2-[2-[[(4S)-4-carboxy-4-[[(4S)-4-carboxy-4-(17-carboxyheptadecanoylamino)butanoyl]amino]butanoyl]amino]-ethoxy]ethoxy]acetyl]amino]ethoxy]ethoxy]acetyl]-[Arg4,Lys7,Gin18,Ile22,Trp30, Leu31]hPYY(4-36) (Compound 28, SEQ ID NO: 13);
   N{alpha-4}-(3-Methylbutanoyl)-N{Epsilon-7}-[(4S)-4-carboxy-4-[[(4S)-4-carboxy-4-(17-carboxyheptadecanoylamino)butanoyl]amino]butanoyl]-[Arg4,Lys7,Gln18,Ile22, Tyr28,Trp30,Leu31]hPYY(4-36) (Compound 29, SEQ ID NO: 15);
   N{alpha-4}-(3-Methylbutanoyl)-N{Epsilon-7}-[2-[2-[2-[[2-[2-[2-[[(4S)-4-carboxy-4-[[(4S)-4-carboxy-4-(17-carboxyheptadecanoylamino)butanoyl]amino]butanoyl]amino]-ethoxy]ethoxy]acetyl]amino]ethoxy]ethoxy]acetyl]-[Arg4Lys7,Gln18,Ile22,Tyr28, Trp30,Leu31]hPYY(4-36) (Compound 30, SEQ ID NO: 15);
   N{alpha-4}-(3-Methylbutanoyl)-N{Epsilon-7}-[2-[2-[2-[[2-[2-[2-[[(4S)-4-carboxy-4-(17-carboxyheptadecanoylamino)butanoyl]amino]ethoxy]ethoxy]acetyl]-amino]ethoxy]ethoxy]acetyl]-[Arg4,Lys7,Gln18,Ile22,Tyr28,Trp30,Leu31]hPYY(4-36) (Compound 31, SEQ ID NO: 15);
   N{alpha-4}-(3-Methylbutanoyl)-N{Epsilon-7}-6-[[(4S)-4-carboxy-4-(17-carboxy-heptadecanoylamino)butanoyl]amino]hexanoyl-[Arg4,Lys7,Gln18,Ile22,Tyr28, Trp30,Leu31]hPYY(4-36) (Compound 32, SEQ ID NO: 15);
   N{alpha-4}-(3-Methylbutanoyl)-N{Epsilon-7}-[(4S)-4-carboxy-4-[[(4S)-4-carboxy-4-(19-carboxynonadecanoylamino)butanoyl]amino]butanoyl]-[Arg4,Lys7,Gln18, Ile22,Tyr28,Trp30,Leu31]hPYY(4-36) (Compound 33, SEQ ID NO: 15);
   N{alpha-4}-(3-Methylbutanoyl)-N{Epsilon-7}-[(4S)-4-carboxy-4-(17-carboxy-heptadecanoylamino)butanoyl]-[Arg4,Lys7,GIn18,Ile22,Ala24,Tyr28,Trp30, Leu31]hPYY(4-36) (Compound 34, SEQ ID NO: 16);
   N{alpha-4}-(3-Methylbutanoyl)-N{Epsilon-7}-[(4S)-4-carboxy-4-(17-carboxy-heptadecanoylamino)butanoyl]-[Arg4,Lys7,Gln18,Gln22,Tyr28,Trp30,Leu31]hPYY(4-36) (Compound 35);
   N{alpha-4}-(3-Methylbutanoyl)-N{Epsilon-7}-[2-[2-[2-[[2-[2-[2-[[(4S)-4-carboxy-4-(17-carboxyheptadecanoylamino)butanoyl]amino]ethoxy]ethoxy]acetyl]-amino]ethoxy]ethoxy]acetyl]-[Arg4,Lys7,Gin18,Gln22,Tyr28,Trp30,Leu31]hPYY(4-36) (Compound 36, SEQ ID NO: 17);
   N{alpha-4}-(3-Methylbutanoyl)-N{Epsilon-7}-[2-[2-[2-[[2-[2-[2-[[(4S)-4-carboxy-4-[[(4S)-4-carboxy-4-(17-carboxyheptadecanoylamino)butanoyl]amino]butanoyl]amino]-ethoxy]ethoxy]acetyl]amino]ethoxy]ethoxy]acetyl]-[Arg4,Lys7,Gin18,GIn22,Tyr28, Trp30,Leu31]hPYY(4-36) (Compound 37, SEQ ID NO: 17);
   N{alpha-4}-(3-Methylbutanoyl)-N{Epsilon-7}-[2-[2-[2-[[2-[2-[2-[[(4S)-4-carboxy-4-(17-carboxyheptadecanoylamino)butanoyl]amino]ethoxy]ethoxy]acetyl]amino]-ethoxy]ethoxy]acetyl]-[Arg4,Lys7,Gln18,Val22,Tyr28,Trp30,Leu31]hPYY(4-36) (Compound 38, SEQ ID NO: 18);
   N{alpha-4}-(3-Methylbutanoyl)-N{Epsilon-7}-[2-[2-[2-[[2-[2-[2-[[(4S)-4-carboxy-4-[[(4S)-4-carboxy-4-(17-carboxyheptadecanoylamino)butanoyl]amino]butanoyl]-amino]ethoxy]ethoxy]acetyl]amino]ethoxy]ethoxy]acetyl]-[Arg4,Lys7,Gln18,Val22, Tyr28,Trp30,Leu31]hPYY(4-36) (Compound 39, SEQ ID NO: 18);
   N{alpha-4}-(3-Methylbutanoyl)-N{Epsilon-7}-[(4S)-4-carboxy-4-(17-carboxy-heptadecanoylamino)butanoyl]-[Arg4,Lys7,Gln18,Val22,Tyr28,Trp30,Leu31]hPYY(4-36) (Compound 40, SEQ ID NO: 18);
   N{alpha-4}-}-(3-Methylbutanoyl)-(N{Epsilon-7}-[(4S)-4-carboxy-4-[[(4S)-4-carboxy-4-(17-carboxy-heptadecanoylamino)butanoyl]amino]butanoyl]-[Arg4,Lys7,Gln18,Val22, Tyr28,Trp30,Leu31]hPYY(4-36) (Compound 41, SEQ ID NO: 18);
   N{alpha-4}-(3-Methylbutanoyl)-N{Epsilon-7}-[(4S)-4-carboxy-4-(17-carboxy-heptadecanoylamino)butanoyl]-[Arg4,Lys7,Thr9,Gin18,Tyr28,Trp30,Leu31]hPYY (4-36) (Compound 42, SEQ ID NO: 7);
   N{alpha-4}-(3-Methylbutanoyl)-N{Epsilon-7}-[(4S)-4-carboxy-4-(17-carboxy-heptadecanoylamino)butanoyl]-[Arg4,Lys7,Thr9,GIn18,Gln22,Tyr28,Trp30,Leu31]hPYY(4-36) (Compound 43, SEQ ID NO: 19);
   N{alpha-4}-(3-Methylbutanoyl)-N{Epsilon-7}-[(4S)-4-carboxy-4-(17-carboxy-heptadecanoylamino)butanoyl]-[Arg4,Lys7,Thr9,Gln18,Val22,Tyr28,Trp30,Leu31]hPYY(4-36) (Compound 44, SEQ ID NO: 20);
   N{alpha-4}-(3-Methylbutanoyl)-N{Epsilon-7}-[(4S)-4-carboxy-4-(17-carboxy-heptadecanoylamino)butanoyl]-[Arg4, Lys7, Thr13, Gln18, Gln22, Tyr28, Trp30,Leu31]hPYY(4-36) (Compound 45, SEQ ID NO: 21);
   N{alpha-4}-(3-Methylbutanoyl)-N{Epsilon-7}-[2-[2-[2-[[2-[2-[2-[[(4S)-4-carboxy-4-(17-carboxyheptadecanoylamino)butanoyl]amino]ethoxy]ethoxy]acetyl]-amino]-ethoxy]ethoxy]acetyl]-[Arg4,Lys7,Thr13,Gln18,Val22,Tyr28,Trp30,Leu31]hPYY(4-36) (Compound 46, SEQ ID NO: 22);
   N{alpha-4}-(3-Methylbutanoyl)-N{Epsilon-7}-[(4S)-4-carboxy-4-(17-carboxy-heptadecanoylamino)butanoyl]-[Arg4,Lys7,Thr13,Gln18,Val22,Tyr28, Trp30,Leu31]hPYY(4-36) (Compound 47, SEQ ID NO: 22);
   N{alpha-4}-(3-Methylbutanoyl)-N{Epsilon-7}-[(4S)-4-carboxy-4-(19-carboxy-nonadecanoylamino)butanoyl]-[Arg4,Lys7,GIn18,Tyr28,Trp30,Leu31]hPYY(4-36) (Compound 48, SEQ ID NO: 4);
   N{alpha-4}-(3-Methylbutanoyl)-N{Epsilon-7}-[(4S)-4-carboxy-4-(17-carboxy-heptadecanoylamino)butanoyl]-[Arg4,Lys7,Gln18,Ile22,Tyr28,Trp30,Leu31]hPYY(4-36) (Compound 49, SEQ ID NO: 15);
   N{alpha-4}-(3-Methylbutanoyl)-N{Epsilon-10}-[(4S)-4-carboxy-4-[[2-[2-[2-[[(4S)-4-carboxy-4-(17-carboxyheptadecanoylamino)butanoyl]amino]ethoxy]ethoxy]-acetyl]amino]butanoyl]-[Arg4,Lys10,Gln18,Glu22,Tyr28,Trp30,Leu31]-PYY(4-36) (Compound 50, SEQ ID NO: 23);
   N{alpha-4}-(3-Methylbutanoyl)-N{Epsilon-10}-[2-[2-[2-[[2-[2-[2-[[(4S)-4-carboxy-4-(17-carboxyheptadecanoylamino)butanoyl]amino]ethoxy]ethoxy]acetyl]-amino]ethoxy]ethoxy]acetyl]-[Arg4,Lys10,Gln18,Glu22,Tyr28,Trp30,Leu31]-PYY(4-36) (Compound 51, SEQ ID NO: 23);
   N{alpha-4}-(3-Methylbutanoyl)-N{Epsilon-10}-[2-[2-[2-[[2-[2-[2-[[(4S)-4-carboxy-4-(17-carboxyheptadecanoylamino)butanoyl]amino]ethoxy]ethoxy]acetyl]amino]-ethoxy]ethoxy]acetyl]-[Arg4,Lys10,Gln18,Glu23,Tyr28,Trp30,Leu31]-PYY(4-36) (Compound 52, SEQ ID NO: 24);
   N{alpha-4}-(3-Methylbutanoyl)-N{Epsilon-10}-[(4S)-4-carboxy-4-[[2-[2-[2-[[(4S)-4-carboxy-4-(17-carboxyheptadecanoylamino)butanoyl]amino]ethoxy]ethoxy]-acetyl]amino]butanoyl]-[Arg4,Lys10,Gln18,Glu23,Tyr28,Trp30,Leu31]-PYY(4-36) (Compound 53, SEQ ID NO: 24);
   N{alpha-4}-(3-Methylbutanoyl)-N{Epsilon-10}-[(4S)-4-carboxy-4-[[2-[2-[2-[[(4S)-4-carboxy-4-(17-carboxyheptadecanoylamino)butanoyl]amino]ethoxy]ethoxy]-acetyl]amino]butanoyl]-[Arg4, Pro9, Lys10,Gln18,Glu22,Tyr28, Trp30,Leu31]-PYY(4-36) (Compound 54, SEQ ID NO: 25);
   N{alpha-4}-(3-Methylbutanoyl)-N{Epsilon-7}-[(4S)-4-carboxy-4-(16-sulfohexadecanoylamino)butanoyl]-[Arg4,Lys7,Gln18,Tyr28,Trp30,Leu31]-PYY(4-36) (Compound 55, SEQ ID NO: 4);
   N{alpha-4}-(3-Methylbutanoyl)-N{Epsilon-10}-[(4S)-4-carboxy-4-[[2-[2-[2-[[(4S)-4-carboxy-4-(17-carboxyheptadecanoylamino)butanoyl]amino]ethoxy]ethoxy]-acetyl]amino]butanoyl]-[Arg4,Pro9,Lys10,Gln18,Tyr28, Trp30,Leu31]-PYY(4-36) (Compound 56, SEQ ID NO: 26);
   N{alpha-4}-(3-Methylbutanoyl)-N{Epsilon-7}-[2-[2-[2-[[(4S)-4-carboxy-4-(17-carboxyheptadecanoylamino)butanoyl]amino]ethoxy]ethoxy]acetyl]-[Arg4,Lys7,Gln18,Val22,Tyr28,Trp30,Leu31]-PYY(4-36) (Compound 57, SEQ ID NO: 18);
   N{alpha-4}-(3-Methylbutanoyl)-N{Epsilon-7}-[2-[2-[2-[[(4S)-4-carboxy-4-(15-carboxypentadecanoylamino)butanoyl]amino]ethoxy]ethoxy]acetyl]-[Arg4,Lys7,Gln18,Tyr28,Trp30,Leu31]-PYY(4-36) (Compound 58, SEQ ID NO: 4);
   N{alpha-4}-(3-Methylbutanoyl)-N{Epsilon-10}-[(4S)-4-carboxy-4-[[2-[2-[2-[[(4S)-4-carboxy-4-(17-carboxyheptadecanoylamino)butanoyl]amino]ethoxy]ethoxy]-acetyl]amino]butanoyl]-[Arg4,Lys10,GIn18,Tyr28,Trp30,Leu31]-PYY-(4-36) (Compound 59, SEQ ID NO: 27);
   N{alpha-4}-(3-Methylbutanoyl)-N{Epsilon-7}-[2-[2-[2-[[(4S)-4-carboxy-4-(17-carboxyheptadecanoylamino)butanoyl]amino]ethoxy]ethoxy]acetyl]-[Arg4,Lys7,Gln18,Ala24,Tyr28,Trp30,Leu31]-PYY(4-36) (Compound 60, SEQ ID NO: 9);
   N{alpha-4}-(3-Methylbutanoyl)-N{Epsilon-7}-[2-[2-[2-[[2-[2-[2-[[(4S)-4-carboxy-4-(15-carboxypentadecanoylamino)butanoyl]amino]ethoxy]ethoxy]acetyl]-amino]ethoxy]ethoxy]acetyl]-[Arg4,Lys7,Pro9,Gln18,Tyr28,Trp30,Leu31]-PYY-(4-36) (Compound 61, SEQ ID NO: 5);
   N{alpha-4}-(3-Methyl butanoyl)-N{Epsilon-7}-[2-[2-[2-[[(4S)-4-carboxy-4-(13-carboxytridecanoylamino)butanoyl]amino]ethoxy]ethoxy]acetyl]-[Arg4,Lys7,Gln18,Tyr28,Trp30,Leu31]-PYY(4-36) (Compound 62, SEQ ID NO: 4);
   N{alpha-4}-(3-Methyl butanoyl)-N{Epsilon-7}-[2-[2-[2-[[2-[2-[2-[[(4S)-4-carboxy-4-(15-carboxypentadecanoylamino)butanoyl]amino]ethoxy]ethoxy]acetyl]-amino]ethoxy]ethoxy]acetyl]-[Arg4,Lys7,Gln18,Tyr28,Trp30,Leu31]-PYY(3-36) (Compound 63, SEQ ID NO: 3);
   N{alpha-4}-(3-Methylbutanoyl)-N{Epsilon-7}-[2-[2-[2-[[2-[2-[2-[[2-[2-[2-[[2-[2-[2-[[(4S)-4-carboxy-4-(17-carboxyheptadecanoylamino)butanoyl]amino]-ethoxy]ethoxy]acetyl]amino]ethoxy]ethoxy]acetyl]amino]ethoxy]ethoxy]acetyl]amino]ethox y]ethoxy]acetyl]-[Arg4,Lys7,Pro9,Gln18,Tyr28,Trp30,Leu31]-PYY(4-36) (Compound 65, SEQ ID NO: 5);
   N{alpha-4}-(3-Methylbutanoyl)-N{Epsilon-7}-[2-[2-[2-[[2-[2-[2-[[(4S)-4-carboxy-4-(16-sulfohexadecanoylamino)butanoyl]amino]ethoxy]ethoxy]acetyl]-amino]ethoxy]ethoxy]acetyl]-[Arg4, Lys7, Pro9,Gln18,Tyr28,Trp30, Leu31]-PYY(4-36) (Compound 66, SEQ ID NO: 5);
   N{alpha-4}-(3-Methylbutanoyl)-N{Epsilon-7}-[2-[2-[2-[[(4S)-4-carboxy-4-(16-sulfohexadecanoylamino)butanoyl]amino]ethoxy]ethoxy]acetyl]-[Arg4,Lys7,Gln18,Tyr28,Trp30,Leu31]-PYY(4-36) (Compound 67, SEQ ID NO: 4);
   N{alpha-4}-(3-Methylbutanoyl)-N{Epsilon-7}-[2-[2-[2-[[2-[2-[2-[[(4S)-4-carboxy-4-(16-sulfohexadecanoylamino)butanoyl]amino]ethoxy]ethoxy]acetyl]-amino]ethoxy]ethoxy]acetyl]-[Arg4,Lys7,Gln18,Tyr28,Trp30,Leu31]-PYY(4-36) (Compound 68, SEQ ID NO: 4);
   N{alpha-4}-(3-Methylbutanoyl)-N{Epsilon-10}-[(4S)-4-carboxy-4-[[2-[2-[2-[[(4S)-4-carboxy-4-(16-sulfohexadecanoylamino)butanoyl]amino]ethoxy]-ethoxy]acetyl]amino]butanoyl]-[Arg4,Pro9,Lys10,Gln18,Tyr28,Trp30,Leu31]-PYY-(4-36) (Compound 69, SEQ ID NO: 26);
   N{alpha-4}-(3-Methylbutanoyl)-N{Epsilon-7}-[(4S)-4-carboxy-4-(13-carboxytridecanoylamino)butanoyl]-[Arg4,Lys7,Pro9,Gln18,Tyr28,Trp30,Leu31]-PYY-(4-36) (Compound 70, SEQ ID NO: 5); and
   N{alpha-4}-(3-Methylbutanoyl)-N{Epsilon-7}-[2-[2-[2-[[(4S)-4-carboxy-4-(13-carboxytridecanoylamino)butanoyl]amino]ethoxy]ethoxy]acetyl]-[Arg4,Lys7,Pro9,Gln18,Tyr28,Trp30,Leu31]-PYY-(4-36) (Compound 71, SEQ ID NO: 5).
27. The pharmaceutical composition according to any one of the preceding embodiments, wherein the PYY compound is N{alpha-4}-(3-Methylbutanoyl)-N{Epsilon-7}-6-[[(4S)-4-carboxy-4-(17-carboxy-heptadecanoylamino)butanoyl]amino]hexanoyl-[Arg4, Lys7,Pro9,Gln18,Tyr28,Trp30, Leu31]hPYY(4-36) (compound 4) 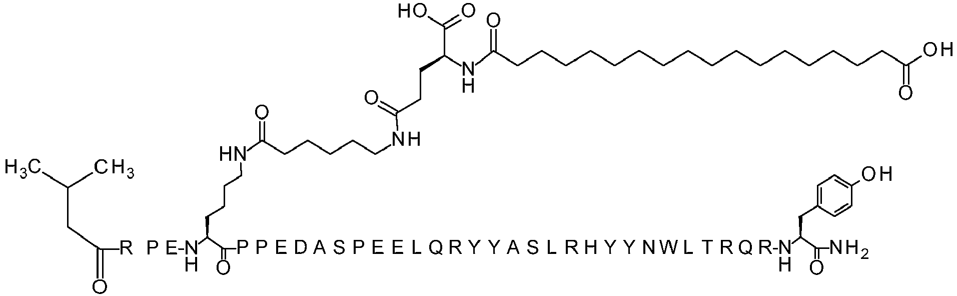
28. The pharmaceutical composition according to any one of the embodiments 1-26, wherein the PYY compound is N{alpha-4}-(3-Methylbutanoyl)-N{Epsilon-7}-6-[[(4S)-4-carboxy-4-(17-carboxy-heptadecanoylamino)butanoyl]amino]hexanoyl-[Arg4,Lys7,Gln18,Ile24, Tyr28,Trp30,Leu31]hPYY(4-36) (compound 20) 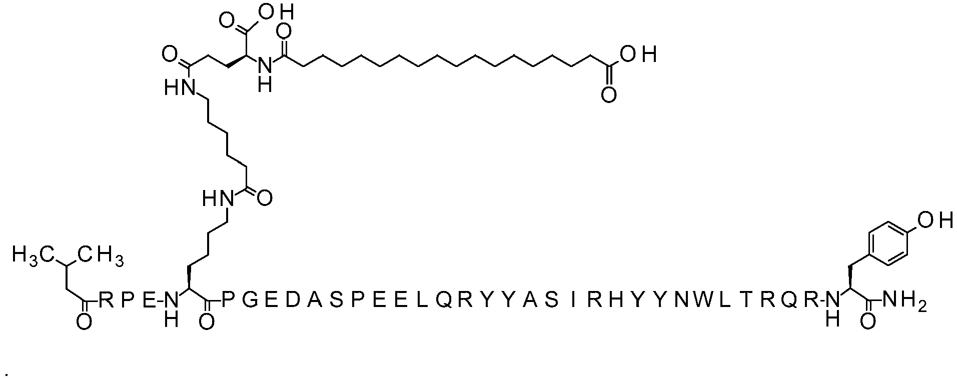
29. The pharmaceutical composition according to any one of the embodiments 1-26, wherein the PYY compound is N{alpha-4}-(3-Methylbutanoyl)-N{Epsilon-7}-6-[[(4S)-4-carboxy-4-(17-carboxy-heptadecanoylamino)butanoyl]amino]hexanoyl-[Arg4,Lys7,Gln18,Ile22,Tyr28, Trp30,Leu31]hPYY(4-36) (compound 32) 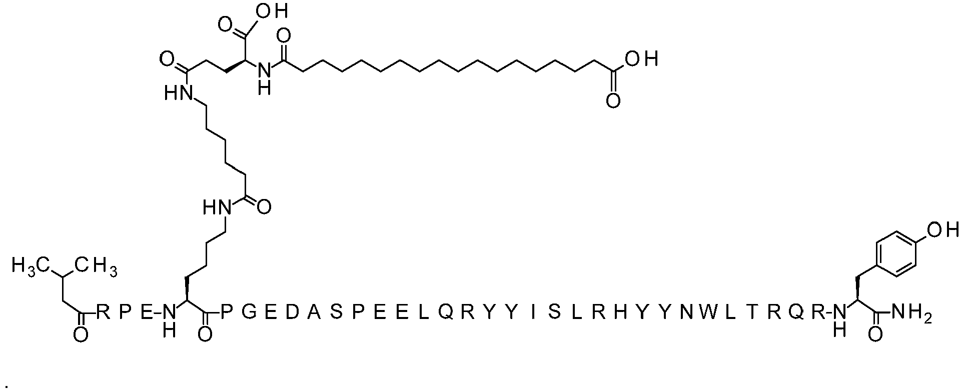
30. The pharmaceutical composition according to any one of the preceding embodiments, wherein the PYY compound is a human Y2 receptor agonist.
31. The pharmaceutical composition according to any one of the preceding embodiments, wherein the PYY compound is a full human Y2 receptor agonist.
32. The pharmaceutical composition according to any one of the preceding embodiments, wherein the PYY compound is a selective human Y2 receptor agonist.
33. The pharmaceutical composition according to any one of the preceding embodiments, wherein the PYY compound is a selective full human Y2 receptor agonist.
34. The pharmaceutical composition according to any one of the preceding embodiments, wherein the PYY compound is capable of activating the human Y2 receptor.
35. The pharmaceutical composition according to any one of the preceding embodiments, wherein the PYY compound is capable of binding to the human Y2 receptor.
36. The pharmaceutical composition according to any one of the preceding embodiments, wherein the composition comprises a GLP-1 receptor agonist.
37. The pharmaceutical composition according to any one of the preceding embodiments, wherein the composition comprises a GLP-1 receptor agonist selected from the group consisting of semaglutide and GLP-1 agonist A.
38. The pharmaceutical composition according to any one of the preceding embodiments, wherein the composition comprises at least one granulate.
39. The pharmaceutical composition according to embodiment 38, wherein the at least one granulate comprises the salt of N-(8-(2-hydroxybenzoyl)amino)caprylic acid.
40. The pharmaceutical composition according to any one of embodiments 38-39, wherein the at least one granulate further comprises a lubricant, such as magnesium stearate.
41. The pharmaceutical composition according to any one of embodiment 38-40, wherein the at least one granulate further comprises the PYY compound and optionally the GLP-1 receptor agonist.
42. The pharmaceutical composition according to any one of embodiments 38-41, wherein the at least one granulate is prepared by dry granulation, such as by roller compaction.
43. The pharmaceutical composition according to any one of embodiments 38-42, wherein the composition comprises an extragranular part.
44. The pharmaceutical composition according to any one of embodiments 38-43, wherein the extragranular part of the composition comprises a lubricant or glidant, such as magnesium stearate and/or the PYY compound.
45. The pharmaceutical composition according to any one of embodiments 38-44, wherein the extragranular part of the composition comprises a lubricant, such as magnesium stearate, the PYY compound and the GLP-1 receptor agonist.
46. A pharmaceutical composition comprising
   a) 0.5-100 mg of a PYY compound and
   b) 20-800 mg, such as 25-600, such as 50-500 mg of a salt of N-(8-(2-hydroxybenzoyl)amino)caprylic acid,
   wherein said salt of N-(8-(2-hydroxybenzoyl)amino)caprylic acid constitutes at least 90 percent (w/w), such as at least 95 percent (w/w) of the excipients of the composition and wherein the PYY compound is compound 4.
47. A pharmaceutical composition comprising
   a) 0.5-50 mg of a PYY compound and
   b) 50-400 mg of a salt of N-(8-(2-hydroxybenzoyl)amino)caprylic acid,
   wherein said salt of N-(8-(2-hydroxybenzoyl)amino)caprylic acid constitutes at least 90 percent (w/w), such as at least 95 percent (w/w) of the excipients of the composition and wherein the PYY compound is compound 4.
48. A pharmaceutical composition comprising
   a) 1-30 mg of a PYY compound and
   b) 75-150 mg of a salt of N-(8-(2-hydroxybenzoyl)amino)caprylic acid,
   wherein said salt of N-(8-(2-hydroxybenzoyl)amino)caprylic acid constitutes at least 90 percent (w/w), such as at least 95 percent (w/w) of the excipients of the composition and wherein the PYY compound is compound 4.
49. A pharmaceutical composition comprising
   a) 1-30 mg of a PYY compound and
   b) 75-125 mg of a salt of N-(8-(2-hydroxybenzoyl)amino)caprylic acid,
   wherein said salt of N-(8-(2-hydroxybenzoyl)amino)caprylic acid constitutes at least 90 percent (w/w), such as at least 95 percent (w/w) of the excipients of the composition and wherein the PYY compound is compound 4.
50. A pharmaceutical composition comprising
   a) 1-30 mg of a PYY compound and
   b) 80-120 mg of a salt of N-(8-(2-hydroxybenzoyl)amino)caprylic acid,
   wherein said salt of N-(8-(2-hydroxybenzoyl)amino)caprylic acid constitutes at least 90 percent (w/w), such as at least 95 percent (w/w) of the excipients of the composition and wherein the PYY compound is compound 4.
51. A pharmaceutical composition comprising
   a) 1-30 mg of a PYY compound and
   b) 200-400 mg of a salt of N-(8-(2-hydroxybenzoyl)amino)caprylic acid,
   wherein said salt of N-(8-(2-hydroxybenzoyl)amino)caprylic acid constitutes at least 90 percent (w/w), such as at least 95 percent (w/w) of the excipients of the composition and wherein the PYY compound is compound 4.
52. A pharmaceutical composition comprising
   a) 1-30 mg of a PYY compound and
   b) 250-350 mg of a salt of N-(8-(2-hydroxybenzoyl)amino)caprylic acid,
   wherein said salt of N-(8-(2-hydroxybenzoyl)amino)caprylic acid constitutes at least 90 percent (w/w), such as at least 95 percent (w/w) of the excipients of the composition and wherein the PYY compound is compound 4.
53. The pharmaceutical composition according to any one of embodiments 46-52, further comprising GLP-1 receptor agonist, e.g. semaglutide or GLP-1 agonist A.
54. The pharmaceutical composition according to embodiment 53, wherein the composition comprises 0.5-50 mg of the GLP-1 receptor agonist, such as 1-25 mg or 1-15 mg pf the GLP-1 receptor agonist.
55. The pharmaceutical composition according to any one of embodiments 46-54, further comprising 1-10 mg lubricant, such as magnesium stearate.
56. The pharmaceutical composition according to any one of embodiments 46-54 further comprising 1-8 mg, such as 2-5 mg or such as 2-3 mg magnesium stearate per 100 mg salt of N-(8-(2-hydroxybenzoyl)amino)caprylic acid.
57. The pharmaceutical composition according to any one of embodiments 46-56, wherein the salt of N-(8-(2-hydroxybenzoyl)amino)caprylic acid is sodium N-(8-(2-hydroxybenzoyl)amino)caprylate (SNAC).
58. A pharmaceutical composition comprising
   a) 0.5-100 mg of a PYY compound and
   b) 20-800 mg, such as 25-600, such as 50-500 mg of a salt of N-(8-(2-hydroxybenzoyl)amino)caprylic acid,
   wherein said salt of N-(8-(2-hydroxybenzoyl)amino)caprylic acid constitutes at least 90 percent (w/w), such as at least 95 percent (w/w) of the excipients of the composition and wherein the PYY compound is compound 32.
59. A pharmaceutical composition comprising
   a) 0.5-50 mg of a PYY compound and
   b) 50-400 mg of a salt of N-(8-(2-hydroxybenzoyl)amino)caprylic acid,
   wherein said salt of N-(8-(2-hydroxybenzoyl)amino)caprylic acid constitutes at least 90 percent (w/w), such as at least 95 percent (w/w) of the excipients of the composition and wherein the PYY compound is compound 32.
60. A pharmaceutical composition comprising
   a) 1-30 mg of a PYY compound and
   b) 75-150 mg of a salt of N-(8-(2-hydroxybenzoyl)amino)caprylic acid,
   wherein said salt of N-(8-(2-hydroxybenzoyl)amino)caprylic acid constitutes at least 90 percent (w/w), such as at least 95 percent (w/w) of the excipients of the composition and wherein the PYY compound is compound 32.
61. A pharmaceutical composition comprising
   a) 1-30 mg of a PYY compound and
   b) 75-125 mg of a salt of N-(8-(2-hydroxybenzoyl)amino)caprylic acid,
   wherein said salt of N-(8-(2-hydroxybenzoyl)amino)caprylic acid constitutes at least 90 percent (w/w), such as at least 95 percent (w/w) of the excipients of the composition and wherein the PYY compound is compound 32.
62. A pharmaceutical composition comprising
   a) 1-30 mg of a PYY compound and
   b) 80-120 mg of a salt of N-(8-(2-hydroxybenzoyl)amino)caprylic acid,
   wherein said salt of N-(8-(2-hydroxybenzoyl)amino)caprylic acid constitutes at least 90 percent (w/w), such as at least 95 percent (w/w) of the excipients of the composition and wherein the PYY compound is compound 32.
63. A pharmaceutical composition comprising
   a) 1-30 mg of a PYY compound and
   b) 200-400 mg of a salt of N-(8-(2-hydroxybenzoyl)amino)caprylic acid,
   wherein said salt of N-(8-(2-hydroxybenzoyl)amino)caprylic acid constitutes at least 90 percent (w/w), such as at least 95 percent (w/w) of the excipients of the composition and wherein the PYY compound is compound 32.
64. A pharmaceutical composition comprising
   a) 1-30 mg of a PYY compound and
   b) 250-350 mg of a salt of N-(8-(2-hydroxybenzoyl)amino)caprylic acid,
   wherein said salt of N-(8-(2-hydroxybenzoyl)amino)caprylic acid constitutes at least 90 percent (w/w), such as at least 95 percent (w/w) of the excipients of the composition and wherein the PYY compound is compound 32.
65. The pharmaceutical composition according to any one of embodiments 58-64, further comprising GLP-1 receptor agonist, e.g. semaglutide or GLP-1 agonist A.
66. The pharmaceutical composition according to embodiment 65, wherein the composition comprises 0.5-50 mg of the GLP-1 receptor agonist, such as 1-25 mg of 1-15 mg pf the GLP-1 receptor agonist.
67. The pharmaceutical composition according to any one of embodiments 58-66, further comprising 1-10 mg lubricant, such as magnesium stearate.
68. The pharmaceutical composition according to any one of embodiments 58-66 further comprising 1-8 mg, such as 2-5 mg or such as 2-3 mg magnesium stearate per 100 mg salt of N-(8-(2-hydroxybenzoyl)amino)caprylic acid.
69. The pharmaceutical composition according to any one of embodiments 58-68, wherein the salt of N-(8-(2-hydroxybenzoyl)amino)caprylic acid is sodium N-(8-(2-hydroxybenzoyl)amino)caprylate (SNAC).
70. The pharmaceutical composition according to any one of the preceding embodiments, wherein the composition is for oral administration.
71. The pharmaceutical composition according to any one of the preceding embodiments, wherein the composition is a solid composition.
72. The pharmaceutical composition according to embodiment 71, wherein the composition is a solid composition, e.g. in the form of a tablet, a capsule or a sachet.
73. A pharmaceutical composition comprising
   a) a PYY compound and
   b) a salt of N-(8-(2-hydroxybenzoyl)amino)caprylic acid,
   wherein the release of the PYY compound reaches 85 percent within 15 minutes or 95 percent within 30 minutes.
74. A pharmaceutical composition comprising
   a) a PYY compound and
   b) a salt of N-(8-(2-hydroxybenzoyl)amino)caprylic acid,
   wherein the dose corrected exposure at t=30 min is increased relative to a test composition 1 herein.
75. A pharmaceutical composition comprising
   a) a PYY compound and
   b) a salt of N-(8-(2-hydroxybenzoyl)amino)caprylic acid,
   wherein the dose corrected exposure (AUC) for t=0-30 min is increased relative to test composition 1 herein.
76. The pharmaceutical composition according to any of embodiments 1-72, wherein
   a) the release of the PYY compound reaches 85 % within 15 minutes,
   b) the release of the PYY compound reaches 95 % within 30 minutes,
   c) the dose corrected exposure at t=30 min is increased relative to test composition 1 herein or
   d) the dose corrected exposure (AUC) for t=0-30 min is increased relative to test composition 1.
77. The pharmaceutical composition according to embodiment 73 or embodiment 76, wherein the dose corrected exposure (AUC) for T=0-30 min is increased at least 1.2 fold, such as at least 1.5 fold, or such as at least 2 fold.
78. The pharmaceutical composition according to any one of the embodiments 73-77, wherein the release is determined as in Assay II herein and/or the dose corrected exposures is determined as in Assay IV.
79. A pharmaceutical composition according to any one of the preceding embodiments for use in medicine.
80. A pharmaceutical composition according to any one of the preceding embodiments for use in treatment and/or prevention of obesity.
81. A pharmaceutical composition according to any one of embodiments 1-78 for use in treatment and/or prevention diabetes, e.g. type 2 diabetes.
82. Use of the composition according to any one of embodiments 1-78, in the manufacture of a medicament for the treatment and/or prevention of eating disorders, such as obesity, e.g. by decreasing food intake, reducing body weight, suppressing appetite, inducing satiety; treating or preventing binge eating disorder, bulimia nervosa, and/or obesity induced by administration of an antipsychotic or a steroid; reduction of gastric motility; delaying gastric emptying; increasing physical mobility; and/or prevention and/or treatment of comorbidities to obesity, such as osteoarthritis and/or urine incontinence.
83. Use of the composition according to any one of embodiments 1-78, in the manufacture of a medicament for the treatment and/or prevention of obesity.
84. Use of the composition according to any one of embodiments 1-78, in the manufacture of a medicament for the treatment and/or prevention of diabetes, e.g. type 2 diabetes.
85. A method of treatment of a subject in need thereof, wherein the method comprises administering a therapeutically effective amount of a composition according to any one of the embodiments 1-78 to said subject.
86. A method of treatment and/or prevention of obesity in a subject, wherein the method comprises administering a therapeutically effective amount of a composition according to any one of embodiments 1-78 to said subject.
87. A method of treatment and/or prevention of diabetes, e.g. type 2 diabetes in a subject, wherein the method comprises administering a therapeutically effective amount of a composition according to any one of the embodiments 1-78 to said subject.
88. A pharmaceutical composition comprising
   a) 0.5-100 mg of PYY compound
   b) a salt of N-(8-(2-hydroxybenzoyl)amino)caprylic acid
   c) a lubricant,

   wherein the PYY compound has a maximum of 10 amino acid modifications as compared to hPYY(3-36) (SEQ ID NO:2) and comprises
      i) lysine at the position corresponding to position 7 or 10 of hPYY(1-36) (SEQ ID NO:1);
      ii) tryptophan at the position corresponding to position 30 of hPYY(1-36) (SEQ ID NO:1);
      iii) leucine at the position corresponding to position 31 of hPYY(1-36) (SEQ ID NO:1);
      iv) tyrosine at the position corresponding to position 28 of hPYY(1-36) (SEQ ID NO:1) and/or isoleucine at the position corresponding to position 22 of hPYY(1-36) (SEQ ID NO:1); and
      v) a modifying group attached to the epsilon amino group of said lysine at the position corresponding to position 7 or 10 of hPYY(1-36) (SEQ ID NO:1),
   wherein said modifying group is defined by A-[B]r-C- or A-[B]r-C-[B]w, wherein
      A- is selected from Chem. 1 and Chem. 2

         Chem. 1: HOOC-(CH2)p-CO-*,

         Chem. 2: HO3S-(CH2)q-CO-*

         wherein p is an integer in the range of 14-18, and q is an integer in the range of 15-17;
      B- is Chem. 3

         Chem. 3: *[NH-CH(COOH)-(CH2)2-CO-]-*,

         r is an integer in the range of 1-3;
         w is an integer in the range of 1-3; and
      C- is absent or selected from Chem. 4 and Chem. 5

         Chem. 4: *[NH-(CH2)2-[O-(CH2)2]s-O-(CH2)t-CO-]u-*

         Chem. 5: *[NH-(CH2)v-CO-]x-*

         wherein s is an integer in the range of 1-3, t is an integer in the range of 1-3, u is an integer in the range of 1-4, v is an integer in the range of 3-7, and x is an integer in the range of 1-3;
   wherein * denotes the points of attachment, and wherein A, B, and C are interconnected via amide bonds and in the sequence indicated via said point of attachments; or a pharmaceutically acceptable salt, amide, or ester of said PYY compound; and
   wherein if the modifying group is A-B-C-B, C cannot be absent.
89. A pharmaceutical composition according to any one of the previous embodiments, wherein A- is selected from Chem. 1 and Chem. 2

   Chem. 1: HOOC-(CH2)p-CO-*,

   Chem. 2: HO3S-(CH2)q-CO-*

   and wherein p is an integer in the range of 16-18, and q is 15.
90. A pharmaceutical composition according to any one of the previous embodiments, wherein A- is Chem. 1

   Chem. 1: HOOC-(CH2)p-CO-*,

   and wherein p is an integer in the range of 14-18.
91. A pharmaceutical composition according to any one of the previous embodiments, wherein A- is Chem. 1

   Chem. 1: HOOC-(CH2)p-CO-*,

   and wherein p is an integer in the range of 16-18.
92. A pharmaceutical composition according to any one of the previous embodiments, wherein B- is Chem. 3

   Chem. 3: *[NH-CH(COOH)-(CH2)2-CO-]-*,

   r is an integer in the range of 1-2;
   w is an interger in the range of 1-2.
93. A pharmaceutical composition according to any one of the previous embodiments, wherein C- is absent or selected from Chem. 4a and Chem. 5a

   Chem. 4a: *[NH-(CH2)2-[O-(CH2)2]2-O-(CH2)2-CO-]u-*

   Chem. 5a: *[NH-(CH2)5-CO-]x-*

   wherein u is an integer in the range of 1-4, and x is an integer in the range of 1-3.
94. A pharmaceutical composition according to to any one of the previous embodiments, wherein the positions corresponding to positions 1 and 2 of hPYY(1-36) (SEQ ID NO:1) are absent.
95. A pharmaceutical composition according to any one of the previous embodiments, wherein the positions corresponding to positions 1-3 of hPYY(1-36) (SEQ ID NO:1) are absent.
96. A pharmaceutical composition according to any one of the previous embodiments, wherein the positions corresponding to positions 1-3 of hPYY(1-36) (SEQ ID NO:1) are absent, and wherein the PYY compound further comprises an N-terminal substituent, wherein the N-terminal substituent is an alkoxy group comprising up to 12 carbon atoms.
97. A pharmaceutical composition comprising
   d) 0.5-100 mg of PYY compound 4, 20 or 32
   e) 20-800 mg, such as 50-500 mg, of a salt of N-(8-(2-hydroxybenzoyl)amino)caprylic acid
   f) a lubricant,
   wherein said salt of N-(8-(2-hydroxybenzoyl)amino)caprylic acid is sodium N-(8-(2-hydroxybenzoyl)amino)caprylate (SNAC) and constitutes at least 90 percent (w/w), such as at least 95 percent (w/w) of the excipients of the composition.
98. A pharmaceutical composition comprising
   a) 0.5-100 mg of PYY compound 4
   b) 20-800 mg, such as 50-500 mg, of a salt of N-(8-(2-hydroxybenzoyl)amino)caprylic acid
   c) a lubricant,
   wherein said salt of N-(8-(2-hydroxybenzoyl)amino)caprylic acid is sodium N-(8-(2-hydroxybenzoyl)amino)caprylate (SNAC) and constitutes at least 90 percent (w/w), such as at least 95 percent (w/w) of the excipients of the composition.
99. A pharmaceutical composition comprising
   a) 0.5-100 mg of PYY compound 20
   b) 20-800 mg, such as 50-500 mg, of a salt of N-(8-(2-hydroxybenzoyl)amino)caprylic acid
   c) a lubricant,
   wherein said salt of N-(8-(2-hydroxybenzoyl)amino)caprylic acid is sodium N-(8-(2-hydroxybenzoyl)amino)caprylate (SNAC) and constitutes at least 90 percent (w/w), such as at least 95 percent (w/w) of the excipients of the composition.
100. A pharmaceutical composition comprising
   a) 0.5-100 mg of PYY compound 32
   b) 20-800 mg, such as 50-500 mg, of a salt of N-(8-(2-hydroxybenzoyl)amino)caprylic acid
   c) a lubricant,
   wherein said salt of N-(8-(2-hydroxybenzoyl)amino)caprylic acid is sodium N-(8-(2-hydroxybenzoyl)amino)caprylate (SNAC) and constitutes at least 90 percent (w/w), such as at least 95 percent (w/w) of the excipients of the composition.
101. A composition according to any one of the previous embodiments, wherein said lubricant is magnesium stearate.
102. A pharmaceutical composition comprising
   a) 0.5-100 mg of a PYY compound
   b) 20-800 mg, such as 50-500 mg, of a salt of N-(8-(2-hydroxybenzoyl)amino)caprylic acid
   c) magnesium stearate,

   wherein the magnesium stearate constitutes 1 to 5 percent (w/w) of the excipients of the composition, and
   wherein said salt of N-(8-(2-hydroxybenzoyl)amino)caprylic acid is sodium N-(8-(2-hydroxybenzoyl)amino)caprylate (SNAC) and constitutes at least 90 percent (w/w), such as at least 95 percent (w/w) of the excipients of the composition.
103. A pharmaceutical composition according to embodiment 102, wherein the magnesium stearate constitutes 1 to 4 percent (w/w) of the excipients of the composition.
104. A pharmaceutical composition according to any one of the embodiments 102-103, wherein the magnesium stearate constitutes 1 to 3 percent (w/w) of the excipients of the composition.
105. A pharmaceutical composition according to any one of the embodiments 102-104, wherein the magnesium stearate constitutes 1 to 2.5 percent (w/w) of the excipients of the composition.
106. A pharmaceutical composition according to any one of the embodiments 102-105, wherein the magnesium stearate constitutes 2 to 3 percent (w/w) of the excipients of the composition.
107. A pharmaceutical composition according to any one of the embodiments 102-106, wherein the magnesium stearate constitutes 2 to 4 percent (w/w) of the excipients of the composition.
108. A pharmaceutical composition according to any one of the embodiments 102-107, wherein the magnesium stearate constitutes 2 to 5 percent (w/w) of the excipients of the composition.
109. A pharmaceutical composition according to any one of the embodiments 102-108, wherein the magnesium stearate constitutes 2 to 2.5 percent (w/w) of the excipients of the composition.
110. A pharmaceutical composition according to any one of the embodiments 102-109, wherein the magnesium stearate constitutes 2.5 percent (w/w) of the excipients of the composition.
111. A pharmaceutical composition according to any one of the embodiments 102-110, wherein the PYY compound has a maximum of 10 amino acid modifications as compared to hPYY(3-36) (SEQ ID NO:2) and comprises
   i) lysine at the position corresponding to position 7 or 10 of hPYY(1-36) (SEQ ID NO:1);
   ii) tryptophan at the position corresponding to position 30 of hPYY(1-36) (SEQ ID NO:1);
   iii) leucine at the position corresponding to position 31 of hPYY(1-36) (SEQ ID NO:1);
   iv) tyrosine at the position corresponding to position 28 of hPYY(1-36) (SEQ ID NO:1) and/or isoleucine at the position corresponding to position 22 of hPYY(1-36) (SEQ ID NO:1); and
   v) a modifying group attached to the epsilon amino group of said lysine at the position corresponding to position 7 or 10 of hPYY(1-36) (SEQ ID NO:1),
   wherein said modifying group is defined by A-[B]r-C- or A-[B]r-C-[B]w, wherein
   A- is selected from Chem. 1 and Chem. 2

      Chem. 1: HOOC-(CH2)p-CO-*,

      Chem. 2: HO3S-(CH2)q-CO-*

      wherein p is an integer in the range of 14-18, and q is an integer in the range of 15-17;
   B- is Chem. 3

      Chem. 3: *[NH-CH(COOH)-(CH2)2-CO-]-*,

      r is an integer in the range of 1-3;
      w is an integer in the range of 1-3; and
   C- is absent or selected from Chem. 4 and Chem. 5

      Chem. 4: *[NH-(CH2)2-[O-(CH2)2]s-O-(CH2)t-CO-]u-*

      Chem. 5: *[NH-(CH2)v-CO-]x-*

      wherein s is an integer in the range of 1-3, t is an integer in the range of 1-3, u is an integer in the range of 1-4, v is an integer in the range of 3-7, and x is an integer in the range of 1-3;

   wherein * denotes the points of attachment, and wherein A, B, and C are interconnected via amide bonds and in the sequence indicated via said point of attachments; or a pharmaceutically acceptable salt, amide, or ester of said PYY compound; and
   wherein if the modifying group is A-B-C-B, C cannot be absent.
112. A pharmaceutical composition according to embodiment 24, wherein A- is selected from Chem. 1 and Chem. 2

   Chem. 1: HOOC-(CH2)p-CO-*,

   Chem. 2: HO3S-(CH2)q-CO-*

   and wherein p is an integer in the range of 16-18, and q is 15.
113. A pharmaceutical composition according to any one of embodiments 111-112, wherein A- is Chem. 1

   Chem. 1: HOOC-(CH2)p-CO-*,

   and wherein p is an integer in the range of 14-18.
114. A pharmaceutical composition according to any one of embodiments 111-113, wherein A- is Chem. 1

   Chem. 1: HOOC-(CH2)p-CO-*,

   and wherein p is an integer in the range of 16-18.
115. A pharmaceutical composition according to any one of embodiments 111-114, wherein B- is Chem. 3

   Chem. 3: *[NH-CH(COOH)-(CH2)2-CO-]-*,

   r is an integer in the range of 1-2;
   w is an interger in the range of 1-2.
116. A pharmaceutical composition according to any one of embodiments 111-115, wherein C- is absent or selected from Chem. 4a and Chem. 5a

   Chem. 4a: *[NH-(CH2)2-[O-(CH2)2]2-O-(CH2)2-CO-]u-*

   Chem. 5a: *[NH-(CH2)5-CO-]x-*

   wherein u is an integer in the range of 1-4, and x is an integer in the range of 1-3.
117. A pharmaceutical composition according to to any one of embodiments 111-116, wherein the positions corresponding to positions 1 and 2 of hPYY(1-36) (SEQ ID NO:1) are absent.
118. A pharmaceutical composition according to any one of embodiments 111-117, wherein the positions corresponding to positions 1-3 of hPYY(1-36) (SEQ ID NO:1) are absent.
119. A pharmaceutical composition according to any one of embodiments 111-118, wherein the positions corresponding to positions 1-3 of hPYY(1-36) (SEQ ID NO:1) are absent, and wherein the PYY compound further comprises an N-terminal substituent, wherein the N-terminal substituent is an alkoxy group comprising up to 12 carbon atoms.
120. A pharmaceutical composition according to embodiment 111, wherein the PYY compound is compound 4, 20 or 32.
121. A pharmaceutical composition according to embodiment 111, wherein the PYY compound is compound 4.
122. A pharmaceutical composition comprising
   a) 0.5-100 mg of PYY compound 4, 20 or 32,
   b) 20-800 mg, such as 50-500 mg, of a salt of N-(8-(2-hydroxybenzoyl)amino)caprylic acid and
   c) magnesium stearate,

   wherein the magnesium stearate constitutes 2.5 percent (w/w) of the excipients of the composition, and
   wherein said salt of N-(8-(2-hydroxybenzoyl)amino)caprylic acid is sodium N-(8-(2-hydroxybenzoyl)amino)caprylate (SNAC) and constitutes at least 90 percent (w/w), such as at least 95 percent (w/w) of the excipients of the composition.
123. A pharmaceutical composition comprising
   a) 0.5-100 mg of PYY compound 4
   b) 20-800 mg, such as 50-500 mg, of a salt of N-(8-(2-hydroxybenzoyl)amino)caprylic acid
   c) magnesium stearate,

   wherein the magnesium stearate constitutes 2.5 percent (w/w) of the excipients of the composition, and
   wherein said salt of N-(8-(2-hydroxybenzoyl)amino)caprylic acid is sodium N-(8-(2-hydroxybenzoyl)amino)caprylate (SNAC) and constitutes at least 90 percent (w/w), such as at least 95 percent (w/w) of the excipients of the composition.
124. A pharmaceutical composition comprising
   a) 1-50 mg of PYY compound 4
   b) 20-800 mg, such as 50-500 mg, of a salt of N-(8-(2-hydroxybenzoyl)amino)caprylic acid
   c) magnesium stearate,

   wherein the magnesium stearate constitutes 2.5 percent (w/w) of the excipients of the composition, and
   wherein said salt of N-(8-(2-hydroxybenzoyl)amino)caprylic acid is sodium N-(8-(2-hydroxybenzoyl)amino)caprylate (SNAC) and constitutes at least 90 percent (w/w), such as at least 95 percent (w/w) of the excipients of the composition.
125. A pharmaceutical composition comprising
   a) 1-30 mg of PYY compound 4
   b) 20-800 mg, such as 50-500 mg, of a salt of N-(8-(2-hydroxybenzoyl)amino)caprylic acid
   c) magnesium stearate,

   wherein the magnesium stearate constitutes 2.5 percent (w/w) of the excipients of the composition, and
   wherein said salt of N-(8-(2-hydroxybenzoyl)amino)caprylic acid is sodium N-(8-(2-hydroxybenzoyl)amino)caprylate (SNAC) and constitutes at least 90 percent (w/w), such as at least 95 percent (w/w) of the excipients of the composition.
126. A pharmaceutical composition comprising
   a) 0.5-100 mg of PYY compound 20
   b) 20-800 mg, such as 50-500 mg, of a salt of N-(8-(2-hydroxybenzoyl)amino)caprylic acid
   c) magnesium stearate,

   wherein the magnesium stearate constitutes 2.5 percent (w/w) of the excipients of the composition, and
   wherein said salt of N-(8-(2-hydroxybenzoyl)amino)caprylic acid is sodium N-(8-(2-hydroxybenzoyl)amino)caprylate (SNAC) and constitutes at least 90 percent (w/w), such as at least 95 percent (w/w) of the excipients of the composition.
127. A pharmaceutical composition comprising
   a) 0.5-100 mg of PYY compound 32
   b) 20-800 mg, such as 50-500 mg, of a salt of N-(8-(2-hydroxybenzoyl)amino)caprylic acid
   c) magnesium stearate,

   wherein the magnesium stearate constitutes 2.5 percent (w/w) of the excipients of the composition, and
   wherein said salt of N-(8-(2-hydroxybenzoyl)amino)caprylic acid is sodium N-(8-(2-hydroxybenzoyl)amino)caprylate (SNAC) and constitutes at least 90 percent (w/w), such as at least 95 percent (w/w) of the excipients of the composition.
128. A pharmaceutical composition comprising
   a) 0.5-100 mg of PYY compound 4
   b) 20-800 mg, such as 50-500 mg, of a salt of N-(8-(2-hydroxybenzoyl)amino)caprylic acid
   c) magnesium stearate,

   wherein the magnesium stearate constitutes 2 to 3 percent (w/w) of the excipients of the composition, and
   wherein said salt of N-(8-(2-hydroxybenzoyl)amino)caprylic acid is sodium N-(8-(2-hydroxybenzoyl)amino)caprylate (SNAC) and constitutes at least 90 percent (w/w), such as at least 95 percent (w/w) of the excipients of the composition.
129. The pharmaceutical composition according to any one of the preceding embodiments, wherein the composition is for oral administration.
130. The pharmaceutical composition according to any one of the preceding embodiments, wherein the composition is a solid composition.
131. The pharmaceutical composition according to embodiment 130, wherein the composition is a solid composition in the form of a tablet, a capsule or a sachet.
132. A pharmaceutical composition comprising
   c) a PYY compound and
   d) a salt of N-(8-(2-hydroxybenzoyl)amino)caprylic acid,
   wherein the release of the PYY compound reaches 85 percent within 15 minutes or 95 percent within 30 minutes.
133. A pharmaceutical composition comprising
   c) a PYY compound and
   d) a salt of N-(8-(2-hydroxybenzoyl)amino)caprylic acid,
   wherein the dose corrected exposure at t=30 min is increased relative to a test composition 1 herein.
134. A pharmaceutical composition comprising
   c) a PYY compound and
   d) a salt of N-(8-(2-hydroxybenzoyl)amino)caprylic acid,
   wherein the dose corrected exposure (AUC) for t=0-30 min is increased relative to test composition 1 herein.
135. The pharmaceutical composition according to any of embodiments 88-131, wherein
   a. the release of the PYY compound reaches 85 % within 15 minutes,
   b. the release of the PYY compound reaches 95 % within 30 minutes,
   c. the dose corrected exposure at t=30 min is increased relative to test composition 1 herein or
   d. the dose corrected exposure (AUC) for t=0-30 min is increased relative to test composition 1.
136. The pharmaceutical composition according to embodiment 132 or embodiment 135, wherein the dose corrected exposure (AUC) for T=0-30 min is increased at least 1.2 fold, such as at least 1.5 fold, or such as at least 2 fold.
137. The pharmaceutical composition according to any one of the embodiments 132-136, wherein the release is determined as in Assay II herein and/or the dose corrected exposures is determined as in Assay IV.
138. A pharmaceutical composition according to any one of the preceding embodiments for use in medicine.
139. A pharmaceutical composition according to any one of the preceding embodiments for use in treatment and/or prevention of obesity.
140. A pharmaceutical composition according to any one of embodiments 88-137 for use in treatment and/or prevention diabetes, e.g. type 2 diabetes.
141. Use of the composition according to any one of embodiments 88-137, in the manufacture of a medicament for the treatment and/or prevention of eating disorders, such as obesity, e.g. by decreasing food intake, reducing body weight, suppressing appetite, inducing satiety; treating or preventing binge eating disorder, bulimia nervosa, and/or obesity induced by administration of an antipsychotic or a steroid; reduction of gastric motility; delaying gastric emptying; increasing physical mobility; and/or prevention and/or treatment of comorbidities to obesity, such as osteoarthritis and/or urine incontinence.
142. Use of the composition according to any one of embodiments 88-137, in the manufacture of a medicament for the treatment and/or prevention of obesity.
143. Use of the composition according to any one of embodiments 88-137, in the manufacture of a medicament for the treatment and/or prevention of diabetes, e.g. type 2 diabetes.
144. A method of treatment of a subject in need thereof, wherein the method comprises administering a therapeutically effective amount of a composition according to any one of the embodiments 88-137 to said subject.
145. A method of treatment and/or prevention of obesity in a subject, wherein the method comprises administering a therapeutically effective amount of a composition according to any one of embodiments 88-137 to said subject.
146. A method of treatment and/or prevention of diabetes, e.g. type 2 diabetes in a subject, wherein the method comprises administering a therapeutically effective amount of a composition according to any one of the embodiments 88-137 to said subject.

### METHODS AND EXAMPLES

### General Methods of Detection and Characterisation

### Assay I: Disintegration test

A disintegration test is performed in an appropriate disintegration apparatus e.g. USP disintegration apparatus to measure the disintegration time of the test compositions *in vitro.*

### Assay II: Dissolution test

A standard dissolution test according to the European Pharmacopeia (Ph Eur 2.9.3) may be performed to measure the release of the PYY compound and SNAC *in vitro.*

A dissolution test is performed in an appropriate dissolution apparatus e.g. USP dissolution apparatus 2. More specifically, an apparatus 2 is used in accordance with United States Pharmacopoeia 35 using a paddle rotation speed of 50 rpm. For testing at pH 6.8, the 500 mL dissolution medium of 0.05 M phosphate buffer is used at a temperature of 37 ± 0.5 °C. Dissolution media has a content of 0.1 % Brij^{®}35. Samples are removed at appropriate intervals and sample content determined using a RP-UHPLC method for dual detection of SNAC and PYY compound.

The sample content is calculated based on the peak area of the PYY compound and SNAC peaks in the chromatogram relative to the peak areas of the PYY compound and SNAC references, respectively. The released amount of PYY compound and SNAC is calculated as percentage of the total content in the test compositions. The total content in the tablets is determined using Assay III.

### Assay III: Analysis of amount of PYY compound and SNAC

For assay analysis the tablets are weighed before extraction of the PYY compound and SNAC. Tablets are dissolved in a relevant amount of 0.05 M phosphate buffer, pH 7.4, with 20% acetonitrile. Extraction time of two hours is used. Samples are centrifuged, and a suitable volume is transferred to HPLC vial. Standards of relevant PYY compound and SNAC are prepared by using the same diluent as for the samples. UHPLC with an UV-detector is used for dual determination of the PYY compound and SNAC content. The tablet content is calculated based on the peak area of the PYY compound and SNAC peaks in the chromatogram relative to the peak areas of the PYY compound and SNAC references, respectively.

### Assay IV: Pharmacokinetic studies in Beagle dogs

Pharmacokinetic (PK) studies in Beagle dogs are conducted to determine the exposure of the PYY compound after peroral administration of different dosage forms.

For the pharmacokinetic studies male Beagle dogs are used, 1 to 5 years of age and weighing approximately 10-12 kg at the start of the studies. The dogs are group housed in pens (12 hours light: 12 hours dark), and fed individually and restrictedly once daily with Royal Canin Medium Adult dog (Royal Canin Products, China Branch, or Brogaarden A/S, Denmark). Exercise and group social are permitted daily, whenever possible. The dogs are used for repeated pharmacokinetic studies with a suitable wash-out period between successive dosings. An appropriate acclimatisation period is given prior to initiation of the first pharmacokinetic study. All handling, dosing and blood sampling of the animals are performed by trained and skilled staff. Before the studies the dogs are fasted overnight and from 0 to 4 h after dosing. Besides, the dogs are restricted to water 1 hour before dosing until 4 hours after dosing, but otherwise have ad libitum access to water during the whole period.

The tablets comprising the PYY compound are administered in the following manner: 10 min prior to tablet administration the dogs are dosed subcutaneously with approximately 3 nmol/kg of SEQ ID NO: 34). The PYY tablets are placed in the back of the mouth of the dog to prevent chewing. The mouth is then closed and tap water is given by a syringe or gavage to facilitate swallowing of the tablet.

### Blood sampling

Blood is sampled at predefined time points for up till 10 hr post dosing to adequately cover the full plasma concentration-time absorption profile of the PYY compound.

For each blood sampling time point approximately 0.8 mL of whole blood is collected in a 1.5 mL EDTA coated tube, and the tube is gently turned to allowing mixing of the sample with the EDTA. Blood samples (for example 0.8 mL) are collected in EDTA buffer (8mM) and then centrifuged at 4°C and 2000G for 10 minutes. Plasma is pipetted into Micronic tubes on dry ice and kept at -20°C until analysis.

Blood samples are taken as appropriate, for example from a venflon in the cephalic vein in the front leg for the first 2 hours and then with syringe from the jugular vein for the rest of the time points (the first few drops are allowed to drain from the venflon to avoid heparin saline from the venflon in the sample).

### Plasma analysis

PYY compound was assayed in dog plasma by plasma protein precipitation and turboflow liquid chromatography tandem mass spectrometry (TF-LC-MS/MS). Calibrators were prepared by spiking blank dog plasma with PYY compound in the range from 0.5 to 200 nM. One volume of calibrators, blank plasma and study samples were precipitated with three volumes of ethanol (with internal standard) and centrifuged. One volume of supernatant was mixed with two volumes of Milli-Q water (with 1% formic acid). The mixture was analysed by TF-LC-MS/MS using a Cyclone turboflow column (0.5 x 50 mm, ThermoFisher Scientific) and an Aeris Peptide 3.6 um XB-C18 analytical column (50 × 2.1 mm, Phenomenex) or a Poroshell SB-C18 2.7 um analytical column (50 × 2.1 mm, Agilent). A gradient elution was conducted using mobile phase A (consisting of milli-Q water with 1% formic acid and 5% methanol/acetonitrile (50/50)) and mobile phase B (consisting of methanol/acetonitrile (50/50) with 1% formic acid and 5% milli-Q water). A TSQ Altis or a QE Plus mass spectrometer (ThermoFisher Scientific) was used as detector in single or parallel reaction monitoring mode (m/z 968.5 to m/z 883.4 or m/z 806.7576, NCE 17). Linear calibration curves (weighed 1/x²) were used for calculating the concentration in the plasma samples. Quality control samples were included. The deviation between nominal and calculated concentration in the calibrators and quality control samples was below 20%.

### General methods for tablet preparation

### Method 1: Dry Granulation

Dry granulation is carried out by roller compaction on a Gerteis MINI-PACTOR. The roller speed is set at 3 rpm and roller compaction force at 6 kN/cm, gap of 1 mm.

Prior to dry granulation SNAC and magnesium stearate and optionally MCC are blended in a suitable blender such as a V-shell blender, Pharmatech MB100.

### Method 2: Tablet compression

Tablets are produced on a Kilian STYL'ONE simulating a Fette 102i or on a Fette 102i mounted with a single set of punches, resulting in 7 mm round, 7.2 × 12 mm or 7.5 × 13 mm oval tablets having no score. Punch size is chosen according to the total tablet weight. The press speed is set to 20rpm. The fill volume is adjusted to obtain tablets having target weights from 107 mg to 403 mg. Compression forces around 7 to 8.5 kN are applied to obtain tablets with a crushing strength of around 56-134 N respective to the tablet size.

Prior to tablet compression the granulates obtained by method 1 are blended with PYY compound and any further excipients on a turbula mixer (7 min, 25 rpm).

### EXAMPLES

### Example 1 - Preparation of compositions

Test compositions were prepared according to table 1 below, comprising a peptide based PYY compound prepared as described in WO2016/198682 (Example 1, compounds 4 and 32). The compounds tested have the following name and structure: N{alpha-4}-(3-Methylbutanoyl)-N{Epsilon-7}-6-[[(4S)-4-carboxy-4-(17-carboxy-heptadecanoylamino)butanoyl]amino]hexanoyl-[Arg4, Lys7,Pro9,Gln18,Tyr28,Trp30, Leu31]hPYY(4-36) (compound 4): 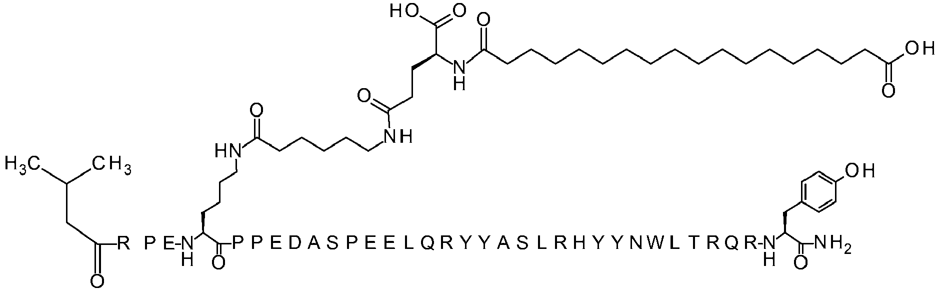 and N{alpha-4}-(3-Methylbutanoyl)-N{Epsilon-7}-6-[[(4S)-4-carboxy-4-(17-carboxy-heptadecanoylamino)butanoyl]amino]hexanoyl-[Arg4,Lys7,Gln18,Ile22,Tyr28, Trp30,Leu31]hPYY(4-36) (compound 32):

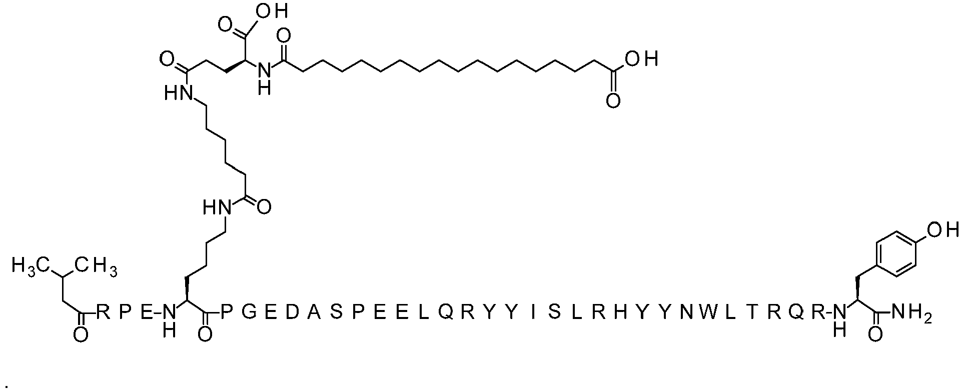

The composition was prepared by using a combination of the methods described herein above. Test composition 1 was produced by granulating a blending of SNAC, magnesium stearate and MCC as described in WO 2013/139694. The granulates were subsequently blended with povidone, the PYY compound and further MCC and magnesium stearate prior to tablet compression (method 2). Test compositions 2, 3 and 4 were prepared by blending of SNAC with magnesium stearate prior to dry granulation (method 1). The obtained granulates where subsequently blended with PYY compound prior to tablet compression (method 2). The compositions are described in table 1.

**Table 1 - composition of PYY compound tablets**

| | **Composition** | **Test 1** | **Test 2** | **Test 3** | **Test 4** |
|---|---|---|---|---|---|
| Granulate | SNAC (mg) | 300 | 100 | 300 | 100 |
| | Magnesium stearate (mg) | 7.7 | 2.6 | 7.7 | 2.6 |
| | MCC (mg) | 57 | - | - | - |
| Extragranular ingredients | Compound 4 (mg) | 3 | 3 | 3 | - |
| | Compound 32 (mg) | - | - | - | 3 |
| | Povidone (mg) | 8 | - | - | - |
| | MCC (mg) | 23 | - | - | - |
| | Magnesium stearate (mg) | 2 | - | - | - |

### Example 2 - Disintegration testing

The objective of the present study was to evaluate the disintegration of the series of the tablet compositions described in Example 1.

Disintegration was measured according to Assay I using a Pharmatech PTZ auto disintegration tester in accordance with European Pharmacopoeia employing automatic detection. Test compositions 1 - 4 were tested in *water R* and considered disintegrated when the automatic detection was deployed. The results are reported as average of 3 tablets.

Table 2 shows the results for tablets prepared according to Example 1 above.

**Table 2. Disintegration times**

| **Composition** | **Test 1** | **Test 2** | **Test 3** | **Test 4** |
|---|---|---|---|---|
| Disintegration time | 12 min 2 s | 3 min 57 s | 7 min 36 s | 5 min 49 s |

The results obtained show that test compositions 2, 3 and 4 display a significantly faster disintegration than observed for test composition 1.

### Example 3 - Dissolution testing

The objective of the present study was to evaluate the dissolution of the series of the tablet compositions described in Example 1.

Dissolution was measured according to Assay II and the amount of SNAC and the PYY compound measured according to Assay III. The released amount of SNAC and PYY compound was calculated as percentages of the actual content in the tablets i.e. 100/300 mg/tablet SNAC and 3 mg/tablet PYY compound.

The content (released amount) of PYY compound is reported as average of 3 tablets.

Table 3 shows the results for tablets prepared according to Example 1 above, wherein the release is presented as "PYY compound in solution (%)" describing the amount of PYY compound in solution after 15, 30 and 60 min relative to the total amount of PYY compound in the tablet at the start of the experiment. The total amount of PYY compound in the tablets were determined according to Assay III.

### Table 3. PYY compound in solution (%)

| **Composition** | **PYY compound in solution (%)** | | |
|---|---|---|---|
| | **15 min** | **30 min** | **60 min** |
| Test 1 | 44 | 67 | 87 |
| Test 2 | 98 | 99 | Full release |
| Test 3 | 92 | Full release | Full release |

The results obtained show that the test composition 2 and 3 display a faster release of the PYY compound compared to what was observed for test composition 1. A significantly faster release of the PYY compound is observed for the early time points, i.e. at 15 and 30 minutes. The difference in release is less significant after 60 minutes. The amount of SNAC in the tablets did not influence the release of the PYY compound substantially i.e. that tablets comprising 100 mg SNAC dissolve as fast as tablets comprising 300 mg SNAC when measured after 15 minutes or later.

Further data obtained after 5, 10, 15, 20, 30, 45 and 60 minutes for test compositions 1-3 are shown in Figure 1, demonstrating that test 2 and test 3 are superior to test 1 at every time point.

### Example 4 - Oral exposure

The objective of this study was to evaluate the oral exposure in beagle dogs of compositions comprising PYY compounds 32 and 4 (Test composition 4 and 3, table 1).

**Table 4. Dose corrected exposure**

| PYY compound | Composition | SNAC amount (mg) | Dose corrected AUC 0-30 min (arbitrary unit) | Dose corrected exposure t=30 min (kg/L) |
|---|---|---|---|---|
| Compound 32 | Test 4 | 100 | 5.05 | 0.268 |
| Compound 4 | Test 3 | 300 | 2.24 | 0.138 |

The data confirmed that the PYY compounds are bioavailable from oral administration in the formulation of the invention.

## Claims

1. A pharmaceutical composition comprising
a) 0.5-100 mg of a PYY compound
b) 20-800 mg, such as 50-500 mg, of a salt of N-(8-(2-hydroxybenzoyl)amino)caprylic acid
c) magnesium stearate,
wherein the magnesium stearate constitutes 1 to 5 percent (w/w) of the excipients of the composition, and
wherein said salt of N-(8-(2-hydroxybenzoyl)amino)caprylic acid is sodium N-(8-(2-hydroxybenzoyl)amino)caprylate (SNAC) and constitutes at least 90 percent (w/w), such as at least 95 percent (w/w) of the excipients of the composition.

2. A pharmaceutical composition according to claim 1, wherein the magnesium stearate constitutes 2 to 4 percent (w/w) of the excipients of the composition.

3. A pharmaceutical composition according to any one of the claims 1-2, wherein the magnesium stearate constitutes 2 to 2.5 percent (w/w) of the excipients of the composition.

4. A pharmaceutical composition according to any one of the claims 1-3, wherein the magnesium stearate constitutes 2.5 percent (w/w) of the excipients of the composition.

5. A pharmaceutical composition according to any one of the claims 1-4,
wherein the PYY compound has a maximum of 10 amino acid modifications as compared to hPYY(3-36) (SEQ ID NO:2) and comprises
i) lysine at the position corresponding to position 7 or 10 of hPYY(1-36) (SEQ ID NO:1);
ii) tryptophan at the position corresponding to position 30 of hPYY(1-36) (SEQ ID NO:1);
iii) leucine at the position corresponding to position 31 of hPYY(1-36) (SEQ ID NO:1);
iv) tyrosine at the position corresponding to position 28 of hPYY(1-36) (SEQ ID NO:1) and/or isoleucine at the position corresponding to position 22 of hPYY(1-36) (SEQ ID NO:1); and
v) a modifying group attached to the epsilon amino group of said lysine at the position corresponding to position 7 or 10 of hPYY(1-36) (SEQ ID NO:1),
wherein said modifying group is defined by A-[B]r-C- or A-[B]r-C-[B]w, wherein
A- is selected from Chem. 1 and Chem. 2
Chem. 1: HOOC-(CH2)p-CO-*,
Chem. 2: HO3S-(CH2)q-CO-*
wherein p is an integer in the range of 14-18, and q is an integer in the range of 15-17;
B- is Chem. 3
Chem. 3: *[NH-CH(COOH)-(CH2)2-CO-]-*,
r is an integer in the range of 1-3;
w is an integer in the range of 1-3; and
C- is absent or selected from Chem. 4 and Chem. 5
Chem. 4: *[NH-(CH2)2-[O-(CH2)2]s-O-(CH2)t-CO-]u-*
Chem. 5: *[NH-(CH2)v-CO-]x-*
wherein s is an integer in the range of 1-3, t is an integer in the range of 1-3, u is an integer in the range of 1-4, v is an integer in the range of 3-7, and x is an integer in the range of 1-3;
wherein * denotes the points of attachment, and wherein A, B, and C are interconnected via amide bonds and in the sequence indicated via said point of attachments; or a pharmaceutically acceptable salt, amide, or ester of said PYY compound; and
wherein if the modifying group is A-B-C-B, C cannot be absent.

6. A pharmaceutical composition according to claim 5, wherein the PYY compound is compound 4, 20 or 32.

7. The pharmaceutical composition according to any one of the preceding claims, further comprising 0.5-20 mg of a GLP-1 receptor agonist selected from semaglutide or GLP-1 agonist A.

8. The pharmaceutical composition according to any one of the preceding claims, wherein the composition is a solid composition, such as a tablet, for oral administration.

9. A pharmaceutical composition according to any one of the preceding claims for use in medicine.

10. A pharmaceutical composition according to any one of the preceding claims for use in the treatment and/or prevention of diabetes and/or obesity.

## Patentansprüche

1. Pharmazeutische Zusammensetzung, umfassend
a) 0,5-100 mg einer PYY-Verbindung
b) 20-800 mg, wie z. B. 50-500 mg, eines Salzes von N-(8-(2-Hydroxybenzoyl)amino)caprylsäure
c) Magnesiumstearat,
wobei das Magnesiumstearat 1 bis 5 Gew.-% der Hilfsstoffe der Zusammensetzung ausmacht, und
wobei das Salz von N-(8-(2-Hydroxybenzoyl)amino)caprylsäure Natrium-N-(8-(2-hydroxybenzoyl)amino)caprylat (SNAC) ist und mindestens 90 Gew.-%, wie z. B. mindestens 95 Gew.-% der Hilfsstoffe der Zusammensetzung, ausmacht.

2. Pharmazeutische Zusammensetzung nach Anspruch 1, wobei das Magnesiumstearat 2 bis 4 Gew.-% der Hilfsstoffe der Zusammensetzung ausmacht.

3. Pharmazeutische Zusammensetzung nach einem der Ansprüche 1-2, wobei das Magnesiumstearat 2 bis 2,5 Gew.-% der Hilfsstoffe der Zusammensetzung ausmacht.

4. Pharmazeutische Zusammensetzung nach einem der Ansprüche 1-3, wobei das Magnesiumstearat 2,5 Gew.-% der Hilfsstoffe der Zusammensetzung ausmacht.

5. Pharmazeutische Zusammensetzung nach einem der Ansprüche 1-4,
wobei die PYY-Verbindung im Vergleich zu hPYY(3-36) (SEQ-ID Nr. 2) maximal 10 Aminosäuremodifikationen aufweist und Folgendes umfasst
i) Lysin an der Position, die Position 7 oder 10 von hPYY(1-36) (SEQ-ID Nr. 1) entspricht;
ii) Tryptophan an der Position, die Position 30 von hPYY(1-36) (SEQ-ID Nr. 1) entspricht;
iii) Leucin an der Position, die Position 31 von hPYY(1-36) (SEQ-ID Nr. 1) entspricht;
iv) Tyrosin an der Position, die Position 28 von hPYY(1-36) (SEQ-ID Nr. 1) entspricht, und/oder Isoleucin an der Position, die Position 22 von hPYY(1-36) (SEQ-ID Nr. 1) entspricht; und
v) eine modifizierende Gruppe, die an der Position, die Position 7 oder 10 von hPYY(1-36) (SEQ-ID Nr. 1) entspricht, an die Epsilon-Aminogruppe des Lysins gebunden,
wobei die modifizierende Gruppe durch A-[B]r-C- oder A-[B]r-C-[B]w definiert ist, wobei
A- ausgewählt ist aus Chem. 1 und Chem. 2
Chem. 1: HOOC-(CH2)p-CO-^{∗},
Chem. 2: HO3S-(CH2)q-CO-^{∗}
wobei p eine ganze Zahl im Bereich von 14 bis 18 ist und q eine ganze Zahl im Bereich von 15 bis 17 ist;
B- Chem. 3
Chem. 3: *[NH-CH(COOH)-(CH2)2-CO-]-*,
r eine ganze Zahl im Bereich von 1 bis 3 ist;
w eine ganze Zahl im Bereich von 1 bis 3 ist; und
C- nicht vorhanden oder ausgewählt ist aus Chem. 4 und Chem. 5
Chem. 4 : *[NH-(CH2)2-[O-(CH2)2]s-O-(CH2)t-CO-]u-*
Chem. 5: *[NH-(CH2)v-CO-]x-*
wobei s eine ganze Zahl im Bereich von 1 bis 3 ist, t eine ganze Zahl im Bereich von 1 bis 3 ist, u eine ganze Zahl im Bereich von 1 bis 4 ist, v eine ganze Zahl im Bereich von 3 bis 7 ist und x eine ganze Zahl im Bereich von 1 bis 3 ist;
wobei * die Bindungsstellen bezeichnet, und wobei A, B und C über Amidbindungen und, in der angegebenen Sequenz, über die Bindungsstellen miteinander verbunden sind; oder ein pharmazeutisch unbedenkliches Salz, Amid oder Ester der PYY-Verbindung; und
wobei, wenn die modifizierende Gruppe A-B-C-B ist, ein Nichtvorhandensein von C unmöglich ist.

6. Pharmazeutische Zusammensetzung nach Anspruch 5, wobei die PYY-Verbindung die Verbindung 4, 20 oder 32 ist.

7. Pharmazeutische Zusammensetzung nach einem der vorhergehenden Ansprüche, die ferner 0,5-20 mg eines GLP-1-Rezeptoragonisten, ausgewählt aus Semaglutid oder GLP-1-Agonist A, umfasst.

8. Pharmazeutische Zusammensetzung nach einem der vorhergehenden Ansprüche, wobei die Zusammensetzung eine feste Zusammensetzung, wie z. B. eine Tablette zur oralen Verabreichung, ist.

9. Pharmazeutische Zusammensetzung nach einem der vorhergehenden Ansprüche zur medizinischen Verwendung.

10. Pharmazeutische Zusammensetzung nach einem der vorhergehenden Ansprüche zur Verwendung bei der Behandlung und/oder Prävention von Diabetes und/oder Adipositas.

## Revendications

1. Composition pharmaceutique comprenant
a) 0,5 à 100 mg d'un composé PYY
b) 20 à 800 mg, par exemple 50 à 500 mg, d'un sel de l'acide N-(8-(2-hydroxybenzoyl)amino)caprylique
c) du stéarate de magnésium,
dans laquelle le stéarate de magnésium constitue 1 à 5 pour cent (p/p) des excipients de la composition, et
dans laquelle ledit sel d'acide N-(8-(2-hydroxybenzoyl)amino)caprylique est le N-(8-(2-hydroxybenzoyl)amino)caprylate de sodium (SNAC) et constitue au moins 90 pour cent (p/p), par exemple au moins 95 pour cent (p/p) des excipients de la composition.

2. Composition pharmaceutique selon la revendication 1, dans laquelle le stéarate de magnésium constitue 2 à 4 pour cent (p/p) des excipients de la composition.

3. Composition pharmaceutique selon l'une quelconque des revendications 1 et 2, dans laquelle le stéarate de magnésium constitue 2 à 2,5 pour cent (p/p) des excipients de la composition.

4. Composition pharmaceutique selon l'une quelconque des revendications 1 à 3, dans laquelle le stéarate de magnésium constitue 2,5 pour cent (p/p) des excipients de la composition.

5. Composition pharmaceutique selon l'une quelconque des revendications 1 à 4,
dans laquelle le composé PYY a un maximum de 10 modifications d'acides aminés comparativement à hPYY(3-36) (SEQ ID NO:2) et comprend
i) lysine à la position correspondant à la position 7 ou 10 de hPYY(1-36) (SEQ ID NO:1) ;
ii) tryptophane à la position correspondant à la position 30 de hPYY(1-36) (SEQ ID NO:1) ;
iii) leucine à la position correspondant à la position 31 de hPYY(1-36) (SEQ ID NO:1) ;
iv) tyrosine à la position correspondant à la position 28 de hPYY(1-36) (SEQ ID NO:1) et/ou isoleucine à la position correspondant à la position 22 de hPYY(1-36) (SEQ ID NO:1) ; et
v) un groupe modificateur fixé au groupe amino epsilon de ladite lysine à la position correspondant à la position 7 ou 10 de hPYY(1-36) (SEQ ID NO:1),
dans laquelle ledit groupe de modification est défini par A-[B]r-C- ou A-[B]r-C-[B]w, dans lequel
A- est choisi parmi la formule chimique 1 et la formule chimique 2
Formule chimique 1 : HOOC-(CH2)p-CO-*,
Formule chimique 2 : HO3S-(CH2)q-CO-^{∗}
dans laquelle p est un entier dans la plage de 14 à 18, et q est un entier compris dans la plage de 15 à 17 ;
B- est de formule chimique 3
Formule chimique 3 : *-[NH-CH(COOH)-(CH2)2-CO-]-*,
r est un entier dans la plage de 1 à 3 ;
w est un entier dans la plage de 1 à 3 ; et
C- est absent ou choisi parmi la formule chimique 4 et la formule chimique 5
Formule chimique 4 : *[NH-(CH2)2-[O-(CH2)2]s-O-(CH2)t-CO-]u-*
Formule chimique 5 : *[NH-(CH2)v-CO-]x-*
dans laquelle q est un entier dans la plage de 1 à 3, t est un entier dans la plage de 1 à 3, u est un entier dans la plage de 1 à 4, et v est un entier dans la plage de 3 à 7, et x est un entier dans la plage de 1 à 3 ;
dans laquelle * désigne les points de fixation, et dans laquelle A, B et C sont interconnectés par l'intermédiaire de liaisons amide et selon la séquence indiquée par l'intermédiaire dudit point de fixation ; ou un sel, amide ou ester pharmaceutiquement acceptable dudit composé PYY ; et
dans laquelle si le groupe modificateur est A-B-C-B, C ne peut pas être absent.

6. Composition pharmaceutique selon la revendication 5, dans laquelle le composé PYY est le composé 4, 20 ou 32.

7. Composition pharmaceutique selon l'une quelconque des revendications précédentes, comprenant en outre 0,5 à 20 mg d'un agoniste du récepteur GLP-1 choisi parmi le sémaglutide ou l'agoniste A de GLP-1.

8. Composition pharmaceutique selon l'une quelconque des revendications précédentes, dans laquelle la composition est une composition solide, par exemple un comprimé pour administration orale.

9. Composition pharmaceutique selon l'une quelconque des revendications précédentes, pour une utilisation en médecine.

10. Composition pharmaceutique selon l'une quelconque des revendications précédentes pour une utilisation dans le traitement et/ou la prévention du diabète et/ou de l'obésité.
